(19) Europäisches Patentamt
European Patent Office
Office européen des brevets

(11) EP 2 851 427 A1

(12) **EUROPEAN PATENT APPLICATION**
published in accordance with Art. 153(4) EPC

(43) Date of publication:
**25.03.2015 Bulletin 2015/13**

(21) Application number: **13804686.7**

(22) Date of filing: **15.05.2013**

(51) Int Cl.:
***C12N 15/13*** *(2006.01)* ***C07K 16/16*** *(2006.01)*
***C12N 15/70*** *(2006.01)* ***G01N 33/53*** *(2006.01)*

(86) International application number:
**PCT/BR2013/000164**

(87) International publication number:
**WO 2013/185193 (19.12.2013 Gazette 2013/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.05.2012 BR 102012011493**

(71) Applicant: **Fundação Butantan**
**São Paulo 05503-900 SP (BR)**

(72) Inventors:
- **KALIL FILHO, Jorge Elias**
  **CEP: 05503-900 São Paulo -SP (BR)**
- **MORO, Ana Maria**
  **CEP: 01411-007 São Paulo - SP (BR)**
- **MIDORI MURATA, Viviane**
  **CEP: 04113-090 São Paulo -SP (BR)**
- **RUMI TSURATA, Lilian**
  **CEP: 04305-000 São Paulo - SP (BR)**
- **COSTA BRAGA SCHMIDT, Mariana**
  **CEP: 04713-020 São Paulo - SP (BR)**

(74) Representative: **Roberts, Mark Peter**
**J A Kemp**
**14 South Square**
**Gray's Inn**
**London WC1R 5JJ (GB)**

# (54) METHOD FOR PRODUCING AND OBTAINING VARIABLE DOMAINS OF ANTI-DIGOXIN MONOCLONAL ANTIBODY FAB FRAGMENT USING THE MOLECULAR BIOLOGY CLONING TECHNIQUE

(57) Method for producing and obtaining variable domains of anti-digoxin monoclonal antibody Fab fragment using the molecular biology cloning technique, and more precisely the present patent of invention relates to a method for producing and obtaining clones of the anti-digoxin antibody Fab fragment using phage display technology and the characterization of the binding thereof to the antigen; the present innovation pertains to the development of the product for therapeutic use having specific potency and a more precise dose for detoxification of patients undergoing treatment with digoxin.

FIG.5

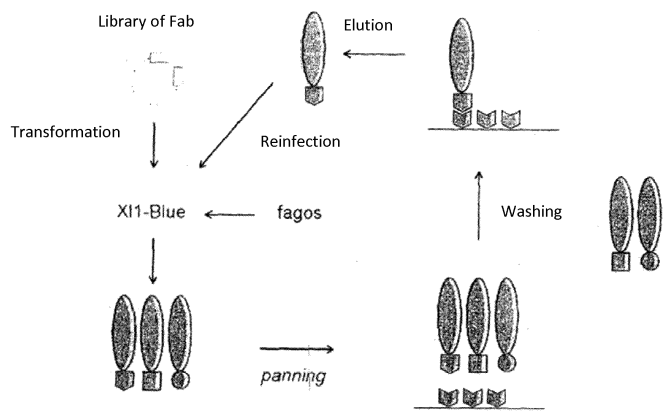


EP 2 851 427 A1

## Description

FIELD OF THE INVENTION

The present patent of invention relates to a method for producing and obtaining clones of the anti-digoxin antibody Fab fragment using phage display technology and the characterization of the binding thereof to the antigen; the present innovation pertains to the development of the product for therapeutic use having specific potency and a more precise dose for detoxification of patients undergoing treatment with digoxin.

BACKGROUND ART

In general, digoxin is a drug used in the treatment of cardiac disorders. It is a digitalis, i.e. a cardiotonic glycoside, and is derived from *Digitalis lanata* plant. It has positive inotropic effect, i.e. it increases the strength of cardiac contraction. Its mode of action is through the reversible binding to $Na^+/K^+$ ATPase. By inhibiting the same, digoxin increases the amount of sodium in the cardiomyocyte. This will stimulate the sodium-calcium pump (exchanges 3 $Na^+$ for 1 $Ca^{2+}$) to expel sodium in exchange for calcium. The resulting increase of calcium within the cell causes an increase in the excitability of the cardiomyocyte, which causes an increase in cardiac contractility.

Digoxin has a narrow therapeutic window, with a therapeutic level very close to the toxic level. To combat its toxic effect, commercially available anti-digoxin polyclonal antibody Fab fragments can be used.

More specifically, digoxin is the cardiac glycoside most widely used in the treatment of congestive heart failure and atrial fibrillation (Figure 1). Digitalis, the group to which digoxin belongs, is the oldest compounds in cardiovascular medicine that are still in use in contemporary clinical practice (EICHHORN, GHEORGHIADE, 2002). Research and experiments with digoxin started more than 200 years ago (WITHERING, 1785) and its applications in patients with heart failure have been described only in the 20th century, describing its ability to increase heart contractility. Digoxin binds to the binding site for cardiac glycosides, present in the $\alpha$ subunit of the Na+ K+-ATPase enzyme, also known as the sodium-potassium pump, and inhibits its activity. This membrane protein is responsible for transporting sodium into the extracellular medium and upon inhibition, it leads to an increase in intracellular sodium. Such increasing stimulates exchanging by the sodium-calcium pump, increasing the intracellular concentration of calcium used by the contractile proteins. As a consequence, there is an increase in the myocardial contraction (EICHHORN, GHEORGHIADE, 2002). Its use in heart failure was approved in 1998 by the Food and Drug Administration (FDA). Its use has decreased with the advent of new therapies such as $\beta$-blockers, angiotensin receptor blockers (ARBs), aldosterone blockers and cardiac resynchronization therapy (CRT). However, it is still used in about 30% of patients with heart failure. It is a low cost drug which is important in developing countries where patients do not have access to sophisticated therapies (GHEORGHIADE et al., 2006).

The concentration of digoxin in serum depends not only depends on the administered dose, but it is also related to interactions with other medicaments and the patient's condition (ANTMAN, SMITH, 1985). The incidence of digoxin poisoning increases in situations where the excretion of digoxin by the kidneys is prevented (SMITH, BUTLER, HABER, 1970). Toxic doses of digoxin may cause serious and fatal arrhythmias (EICHHORN, GHEORGHIADE, 2002).

The use of digitalis has been facilitated by the generation of antibodies used to monitor drug concentrations in the serum of patients with the objective of maintaining safe levels. Digoxin is not antigenic because it is a small molecule (780.92 Daltons) and must be covalently linked to appropriate immunogenic proteins (carriers) to produce specific anti-digoxin antibodies. Digoxin was conjugated to BSA in 1967 to obtain specific antibodies in rabbits (BUTLER, CHEN, 1967). In 1970, Smith et al. reported the high affinity and specificity of antibody populations selected for haptens of cardiac glycosides. Digoxin-specific antibody Fab fragments (Fragment antigen binding) obtained in sheeps have demonstrated ability to reverse digoxin poisoning (SMITH et al., 1976). A study carried out in dogs showed that intravenous administration of purified portion of sheep anti-digoxin polyclonal antibody Fab fragment can quickly reverse cardiotoxicity by binding to free digoxin in plasma and making the redistribution of the drug from tissues back to blood circulation. Fab fragments are relatively fast excreted in urine; therefore, high affinity Fab fragments that retain binding with the drug may provide a clearance route of the drug, as well as a means of neutralizing it (BUTLER et al., 1977). Potassium therapies and adrenergic beta blocking agents have been used as an alternative to combat digitalis intoxication, but the administration of antibody Fab fragments is the main therapy to reverse the intoxication (ANTMA et al., 1990). A retrospective study with 141 patients intoxicated by digoxin evaluated the results of 66 patients treated with Fab fragments and suggested its use as unique first-line therapy due to the low mortality rate of patients treated by the fragments (LAPOSTOLLE et al., 2008).

The anti-digoxin polyclonal antibody Fab fragment generated in sheep is being manufactured by only one company (Protherics) with the name DigiFab and received FDA approval in 2001.

In Brazil, the Fab anti-digoxin antibody can be imported according to Resolution RDC No. 28 of May 9, 2008, of the National Health Surveillance Agency, which permits importing the drugs comprised in the list of exceptionally allowed drugs solely intended to hospital use or prescription use, the importation of which is bounded to a specific hospital authority and/or representative civil organization for its exclusive use and is not intended for resale or trade.

The use of Fab fragments in cases of poisoning by digoxin is safe and effective, but neutralization with Fab fragments is an expensive therapy, only being used when other treatment options seem to fail. Its high cost also limits its availability to all hospitals or countries. (ANDRÉS, 2000; FLANAGAN, JONES, 2004).

Namely, the antibodies are glycoproteins composed of two identical light chains (LC) of approximately 24 kDa each and two identical heavy chains (HC) of 55 to 70 kDa each. A light chain is covalently linked to a heavy chain by a disulfide bridge, while the two heavy chains are linked together by disulfide bonds. The carboxyterminal regions of the heavy chains have effector functions. The light and heavy chains have an amino-terminal variable region (VL - variable light and VH - variable heavy) involved in recognition of antigens. Each variable region of the heavy and light chains contains three hypervariable regions, also called complementarity determining regions (CDRs), named after the amino terminal portion as CDR1, CDR2 and CDR3, CDR3 being the most variable (ABBAS LICHTMAN, 2005). More conserved sequences called framework regions: FR1, FR2, FR3 and FR4 (TONEGAWA, 1983) are present amongst these regions. Mice have two families of light chain genes, lambda ($\lambda$) and kappa ($\kappa$) which differ by their constant regions. Chain $\lambda$ constitutes only about 5% of the total light chain genes, besides being much less heterogeneous than $\kappa$ chain or heavy chain. (TONEGAWA, 1983). The heavy chain constant region is divided into five classes, IgG, IgA, IgM, IgD, and IgE. IgG having a molecular weight of 150 kDa, is the most abundant in mammalian serum. In humans, it is subdivided into IgG1, IgG2, IgG3 and IgM4. IgG1 is the most commonly found in human serum (ABBAS, LICHTMAN, 2005) and it has well defined structure with two disulfide bonds linking the heavy chains in the hinge region (between CH1 and CH2).

In 1975, Kohler and Milstein developed a technology for the production of monoclonal antibodies (MoAbs), which earned them the Nobel Prize in Physiology and Medicine in 1984. The technique consists in generating hybridomas, which are cells originating from the fusion of myeloma cells capable of indefinite reproduction, to B lymphocytes removed from the spleen of a previously immunized animal, which secrete antibodies (KÖHLER, MILSTEIN, 1975). In 1986, the FDA approved the first MoAb for therapeutic use in humans (OKT3). Therapeutic antibodies and fragments thereof, obtained by all available technologies represent the most promising market among biopharmaceuticals generating $ 16.921 billion in the USA in 2009, (AGGARWAL, 2010). Many murine monoclonal antibodies have been produced for diagnosis or treatment of human diseases. However, its use is limited since it has short half-life in serum and due to its high immunogenicity, which may cause a reaction known as human anti-mouse antibody (HAMA). To overcome these problems, research to generate less immunogenic antibodies such as chimeric, humanized, fully human antibodies and antibody fragments has started (MORO, RODRIGUES, 2001).

Antibody fragments are more advantageous for certain clinical applications that do not rely on whole antibody, maintain the antigen binding sites, are less immunogenic than whole IgG, diffuse better in the interstitial space, are easily eliminated via glomerular filtration and are more stable to storage than IgG (FLANAGAN, JONES, 2004). They can be obtained by immunoglobulin digestion with pepsin enzyme that produces two Fab fragments linked by disulfide bridge, divalent F(ab')2, weighing approximately 110 kDa and fragmented Fc portion, or by papain enzyme that produces two monovalent Fab fragments having a molecular weight of 50 kDa and the entire Fc portion (Figure 2). The commercially available anti-digoxin Fab fragments were obtained by digestion of IgG using the papain enzyme. Antibody fragments may also be obtained by expression in *Escherichia coli,* having the advantage of obtaining in large quantities at low costs. Recombinant DNA technology also offers the opportunity to introduce mutations in the gene sequences that can be advantageous. In the case of Fab fragments, it can increase the expressed amount, stability, solubility and facilitate humanization and affinity maturation (KWONG, RADER, 2009).

PHAGE DISPLAY TECHNOLOGY

The phage display technology comprises a method widely used in the generation of antibody fragments. It was introduced in 1985 when George Smith demonstrated that the correlation between genotype and phenotype could be established in filamentous bacteriophage (phages) (Figure 3). Smith showed that exogenous DNA fragments could be inserted into gene III, which encodes the capsid protein (pIII) of these phage, creating a fusion protein. The exogenous peptides exposed on the surface of phages could be selected by affinity for the specific antibody, allowing them to be enriched in relation to the original peptide by a process usually called panning.

In 1989, Huse et al. reported the generation of a library of Fab fragments in lambda phage from the random combination of heavy and light chains of the murine antibody as a method that could replace the hybridoma technology. The variable immunoglobulin genes can be amplified from hybridoma or B lymphocyte cells by using oligonucleotide universal primers and polymerase chain reaction (PCR), allowing cloning into expression vectors (ORLANDI et al., 1989). McCafferty et al. (1990) showed that the complete variable domains of an antibody could be displayed as a single chain variable fragment (scFv) fused to pIII on the surface of phage fd, allowing the phage selection by affinity to the antigen, thus initiating the use of phage display technology for the production of antibodies. The construction of the vector pComb3 allowed Fab fragments fused to pIII to be exposed on the surface of phage M13 (BARBAS et al., 1991). The technology allows the selection of a phage clone displaying antibody fragments having high affinity and specificity for a particular antigen within a phage library built by rearranging the variable domains (CLACKSON et al., 1991). The construction of an antibody by phage display technology begins with the construction of an immunoglobulin library which can be generated from immunized animals, non-immunized animals or a synthetic library (STRACHAN et al., 2002).

Class Ff filamentous phages (f1, fd and M13) have 11 genes (I - XI) and five of them encode capsid proteins that can be fused to the recombinant proteins of interest with varying degree of success, pIII being the most used protein for phage display. Proteins may also be exposed as phage small particles called phagemids, which are plasmids that carry the gene of the recombinant capsid protein and contains origin of replication of phage M13 (BARBAS et al., 2001). The phagemid DNA may be packaged into viral particles with the aid of a helper phage that provides all proteins and enzymes of the wild phage which are necessary for phage replication. The capsid fusion protein is exposed as particles containing the phagemid and helper phage genomes. These particles containing both genomes can be selected by selection markers (BARBAS et al., 1991). The helper phage has a defective replication origin and its genome is inefficiently packaged if compared to the phagemid (BARBAS et al., 2001).

After selection and characterization of the monoclonal antibody, the phage display technology allows the manipulation of the antibody genes by the generation of mutants. This enables the selection of new antibody variants, some of them having enhanced affinity and specificity in relation to the original clone (KRYKBAEV et al., 2002).

Phage display is a technology available in Molecular Biology laboratories. However, it is not an ordinary technology and several details need to be worked in order to get a good result.

Due to the advantages of phage display technology and the importance of anti-digoxin antibodies in cases of poisoning and due to the fact that its commercial production is foreign and expensive, applicants believe in the need of developing and producing this drug in Brazil. The anti-digoxin antibody produced by phage display technology is an alternative for the treatment of digitalis poisoning, since it can significantly decrease drug costs, which are considered high because it is an imported drug.

## PRIOR ART ANALYSIS

A brief search on patent databases regarding the subject "monoclonal anti-digoxin" resulted in some documents, such as the North American US 4803167 (1989 - The General Hospital Co.) which discloses a hybridoma culture and high affinity monoclonal antidigoxin antibodies produced by somatic cell fusion.

The document CN 1375505 (2002 - GEN HOSPITAL PLA) relates to a gene engineering human monoclonal digoxin-resisting single-chain antibody. Its structure is: sequence signature: length: 684 bp, class type, nucleic acid, number of chains: double-stranded geometric structure; linearity; molecule type: DNA (genome); source: positive cloned variable region respectively belongs to human antibody VH5 and V lambda I subgroup; the systematic name of host containing carrier is *Escherichia coli,* CGMCC, No.0542. Said invented gene engineering human monoclonal digoxin-resisting sigla-chain antibody is used for detecting the application of digoxin concentration in patient's serum and detecting the toxic effect resulted from antagonist against digoxin of patient poisoned by digoxin.

## BRIEF DESCRIPTION OF THE INVENTION

In a broader view, the present patent of invention relates to a method for producing and obtaining clones of the anti-digoxin antibody Fab fragment using phage display technology and the characterization of the binding thereof to the antigen.

More specifically, the invention has the following objectives:

- Extracting total RNA from anti-digoxin hybridoma and amplifying LC and HC genes, Fd portion (VH and CH1) of immunoglobulins by PCR;
- Building a combinatorial library of Fab fragments in the phage display vector;
- Selecting anti-digoxin clones by binding to the antigen;
- Expressing soluble anti-digoxin Fab fragments, and
- Characterizing the binding of anti-digoxin Fab fragments of the clones to the antigen.

Materials and methods specially developed for obtaining clones of anti-digoxin antibody Fab fragment using the phage display technology were employed. A number of variants were obtained, and four of them were characterized by binding to digoxin conjugated to BSA (Dig-BSA). Contrary to imported commercial products, this is a monoclonal antibody and is not produced in animals. Thus, the present invention is dedicated, in particular, to the development of a product for therapeutic use having specific potency and a more precise dose for detoxification of patients undergoing treatment with digoxin.

DESCRIPTION OF THE DRAWINGS

In order to complement the present description so as to obtain a better understanding of the features of the present invention and according to a preferred practical embodiment thereof, the description has a set of drawings attached, which by way of non-exhaustive example represents its operation:

Figure 1 - Chemical structure of digoxin (C41H64014), a cardiac glycoside having 780.92 Da;
Figure 2 - Structure of the IgG molecule and fragments thereof that maintains antigen binding sites; immunoglobulin fragments shown: Fab, F(ab')2.
Figure 3 - Schematic representation of Ff class filamentous bacteriophage and its capsid proteins; the capsid proteins shown may be used for expressing recombinant proteins in the form of a fusion protein by phage display technology;
Figure 4 - Schematic representation of the phagemid vector pComb3XTT used in the phage display system; the vector has the LC and HC genes of the human Fab fragment for tetanus toxin -TT‖. The light chain was cloned into the restriction sites SacI and XbaI and the heavy chain of the Fab fragment was cloned into the restriction sites XhoI and SpeI;
Figure 5 - Schematic representation of the main steps of phage display and panning; the library of Fab fragments was transformed into *E. coli* cells XL1-Blue infected by the helper phage, and the phages displaying anti-digoxin Fab fragments were selected by panning rounds through Dig-BSA binding immobilized on the plate. Phages that did not bind were discarded by washing and phages that bound were eluted and used to re-infect *E. coli* XL1-Blue.
Figure 6 - Dig-BSA antigen binding profile to anti-digoxin IgG present in the hybridoma culture supernatant by ELISA test, wherein the Dig-BSA conjugate was immobilized at four concentrations: 1, 2, 4 and 8 $\mu$g/mL. Anti-digoxin hybridoma culture supernatant was used as primary antibody. Anti-mouse IgG peroxidase conjugate was used as secondary antibody;
Figure 7 - Electrophoretic profile of anti-digoxin IgG purified through affinity chromatography by protein A, wherein 12% SDS-PAGE gel was stained by using Coomassie. M: molecular weight marker LMW (Amersham), 1: Unreduced purified IgG, 2: Purified IgG reduced with $\beta$-ME;
Figure 8 - Purified anti-digoxin IgG binding profile to Dig-BSA immobilized in different concentrations to standardize ELISA tests, wherein the Dig-BSA conjugate was immobilized at six concentrations: 0.5, 1, 2, 4, 8 and 10 $\mu$g/mL. The anti-digoxin IgG purified from the hybridoma was used as primary antibody at an initial concentration of 500 ng/mL. Anti-mouse IgG peroxidase conjugate was used as secondary antibody;
Figure 9 - Products of amplification of the cDNAs of anti-digoxin hybridoma by using oligonucleotides of murine immunoglobulin wherein the amplifications were carried out by PCR and analyzed by electrophoresis in 1.5% agarose gel. (A) Products of the HC gene amplifications using oligonucleotide 3'MoIgG1 and the seven oligonucleotides 5'. M: molecular mass marker of 100 pb (Invitrogen), 1: oligonucleotide 5' 1A+1B, 2: 2a, 3: 2B, 4: 2C: 5: 3A, 6: 3B+3C and 7: 3D. (B) Products of the LC gene amplifications using the oligonucleotide Mok 3' and the six oligonucleotides 5'. M: molecular weight marker, 100 bp (Promega), 1: oligonucleotide 5' $\kappa$1, 2: $\kappa$2, 3: $\kappa$3, 4: $\kappa$4, 5: $\kappa$5, and 6: $\kappa$6. The oligonucleotide sequence is shown in Table 1.;
Figure 10 - Restriction analysis of LC library built by cloning the repertoire of LC genes in vector pComb3X to verify the presence of the LC insert; ten randomly selected clones (numbered 1-10) of the LC library and the vector pComb3XTT, used as a control (C), were digested with the restriction enzymes SacI and XbaI and analyzed by electrophoresis on a 1% agarose gel. M: molecular weight marker, 1 kb (Invitrogen).
Figure 11 - Restriction analysis of combinatorial Fab fragment library built in vector pComb3X to check the presence of LC and HC inserts; ten randomly selected clones (numbered 1 to 10) of the combinatorial library were digested with the restriction enzymes SpeI and XhoI to verify the presence of the HC insert (A) and digested with SacI and XbaI to verify the presence of the LC insert (B). The digested samples were subjected to electrophoresis on 1% agarose gel, M: molecular weight marker, 1 kb (Invitrogen);
Figure 12 - Restriction analysis of the diversity of combinatorial library built in vector pComb3X by digestion with restriction enzyme BstNI; the ten randomly selected clones analyzed in Figure 11 and the vector pComb3XTT, used as a control (C), were subjected to electrophoresis on a 3% agarose gel after digestion with the restriction enzyme BstNI. M: molecular weight marker, 100 bp (Promega);
Figure 13 - Restriction analysis of anti-digoxin Fab fragment library after 3 rounds of phage display followed by panning to check the presence of LC and GH genes; ten randomly selected clones (numbered 1 to 10) from the library and the vector pComb3XTT, used as a control (C), were digested with the restriction enzymes SpeI and XhoI to verify the presence of the HC insert (A) and digested with SacI and XbaI to verify the presence of the LC (B) gene and analyzed by electrophoresis on a 1% agarose gel. M: molecular weight marker, 1 kb (Invitrogen);
Figure 14 - Restriction analysis of the diversity of anti-digoxin Fab fragment library after 3 rounds of phage display followed by panning by digestion with the restriction enzyme BstNI; the ten clones (numbered 1 to 10) refer to the clones analyzed in Figure 13, were subjected to electrophoresis on a 3% agarose gel. M: molecular weight marker, 100 bp (Promega);

Figure 15 - A symbolic representation of the amino acid sequences deduced from the LC of clones 1, 2, 9 and 10 selected after phage display; the amino acid residues were replaced by symbols in FR1 and CDR2 regions to highlight the different sequences. The other regions have sequences which are identical to each other.

Figure 16 - Electrophoretic profile of clones 1, 2, 9 and 10 digested with the restriction enzymes NheI and SpeI to remove gene III encoding the pIII protein; bands of approximately 4 kb (vectors) and 612 bp (gene III) were separated by electrophoresis on a 1% agarose gel and the vector band was gel purified to obtain the vector for expression of soluble Fab fragment. M: molecular weight marker, 1 kb (Invitrogen);

Figure 17 - Western blotting of crude extracts containing anti-digoxin Fab fragments of clones 1, 2, 9 and 10 expressed in *E. coli* XL1-blue; the extracts were 1/2 diluted and applied to 12% polyacrylamide gel in nonreduced form. After electrophoresis, the gel was transferred to a PVDF membrane. The membrane was incubated with anti-mouse IgG antibody specific for F(ab')2 fragment conjugated to peroxidase and detected with the ECL system;

Figure 18 - Immunodetection of the antigen Dig-BSA and BSA by anti-digoxin Fab fragments expressed in *E. coli* by 4 clones selected after phage display; (A) Analysis by SDS-PAGE 7.5% of Dig-BSA and BSA (control) in the reduced form. After electrophoresis, the gel was stained with silver. M: HMW marker (GE Healthcare), 1: Dig-BSA 0.025 mg/mL, 2: Dig-BSA 0.0125 mg/mL, 3: Dig-BSA 0.0063 mg/mL, 4: BSA 0.01 mg/mL. (B) Western blotting to verify anti-digoxin Fab fragments of crude extracts from the 4 clones binding to Dig-BSA antigen. Four gels with the same samples used in (A) were transferred to PVDF membranes and each membrane was incubated with the crude extract of *E. coli* containing anti-digoxin Fab fragments from each of the four clones. Then the membranes were incubated with anti-mouse IgG antibody specific for F(ab')2 fragment conjugated to peroxidase and developed with the ECL system.

Figure 19 - Binding profile of the anti-digoxin Fab fragments expressed in *E. coli* by the 4 clones to Dig-BSA and BSA by ELISA test; the microplate was sensitized with Dig-BSA or BSA at 4 μg/mL. The Fab fragments present in the crude extract of *E. coli* cultures were 1:2 serially diluted, beginning with the antibody concentration of 50 ng/mL. The secondary antibody used was anti-mouse IgG specific for F(ab')2 fragment conjugated to peroxidase, and

Figure 20 - BIAcore sensorgram of the binding assay of Fab fragments expressed in *E. coli* by using the 4 clones to Dig-BSA antigen; Dig-BSA was immobilized on a CM5 sensor chip to 9259.8 RU. Crude extracts from clones containing the Fab fragments expressed in *E. coli* were injected at a concentration of 2.0 μg/mL. The relative response was measured 5 seconds after the application of the analyte at the point indicated by the red arrow (clone 1 - 495.9 RU; clone 2 - 476.3 RU; clone 9 - 206.9 RU; clone 10 - 1,023.5 RU).

## DETAILED DESCRIPTION OF THE INVENTION

According to the illustrations, the present invention refers to a METHOD FOR PRODUCING AND OBTAINING MONOCLONAL ANTI-DIGOXIN ANTIBODY FAB FRAGMENT USING THE MOLECULAR BIOLOGY CLONING TECHNIQUE, more particularly to a method for producing and obtaining clones of anti-digoxin antibody Fab fragment using the phage display technology and the characterization of its antigen binding, which comprises the following steps:

Step 1) - Obtaining and characterizing bovine albumin conjugated with digoxin (Dig-BSA)

Phase 1.1 - Obtaining Dig-BSA conjugates;
Phase 1.2 - Characterizing Dig-BSA conjugates;

Step 2) - Preparing the anti-digoxin monoclonal antibody; Phase 2.1 - Purifying the monoclonal anti-digoxin antibody;
Step 3) - Building the Fab library in pComb3X phagemid from anti-digoxin hybridomas

Phase 3.1 - Obtaining cDNA from anti-digoxin hybridomas; Phase 3.2 - Amplifying genes of LC and of Fd portion of HC;
Phase 3.3 - Obtaining competent cells and vector;
Phase 3.4 - Cloning the LC repertoire of genes in vector pComb3X;
Phase 3.5 - Cloning the HC repertoire of genes in the LC library;

Step 4) - Phage display and enrichment of Fab library;

Phase 4.1 - Preparing helper phage;
Phase 4.2 - Generating anti-digoxin Fab phage library (phage display);

Step 5) - Expressing soluble Fab fragments;

Phase 5.1 - Quantifying soluble Fab fragments by sandwich enzyme-linked immunosorbent assay (ELISA);

Step 6) - Characterizing crude extracts containing Fab fragments of clones obtained by phage display

Phase 6.1 - Soluble Fab fragment antigen binding assay by ELISA test;
Stage 6.2 - Polyacrylamide gel electrophoresis with sodium dodecyl sulphate (SDS-PAGE);
Phase 6.3 - Western blotting assay;
Phase 6.4 - Affinity analysis using BIAcore T100.

In order to better define each step and its respective phase, the method is described below in details, which may be accompanied by the drawings which have already been listed:

Step 1) Obtaining and characterizing bovine albumin conjugated with digoxin (Dig-BSA)

Phase 1.1 Obtaining Dig-BSA conjugates

The protocol was based on the technique described by Erlanger and Beiser (1964). Forty four milligrams of digoxin were resuspended in 2 mL of 95% etanol, 2 mL of NaIO4 0.1 M were added and the solution was allowed to stand at room temperature for 30 minutes. After 30 minutes of interaction, 60 μL of glycerol 1 M were added to inactivate the excess periodate and the solution was stirred for 5 minutes. Fifty six milligrams of BSA (Sigma) were dissolved in 2 mL of PBS and the pH was adjusted to 9.5 with 5% Na2CO3 solution. The digoxin solution oxidized with periodate was slowly and dropwise added to the BSA solution, keeping the pH between 9.3 and 9.5 by the addition of 5% Na2CO3. The solution remained under stirring for 1 hour at room temperature, keeping the pH stable for 30 minutes in the range of 9.3 to 9.5. 6 mg of NaBH4 dissolved in 2 mL of immediately prepared deionized water were added. The solution was covered with parafilm (H2 release could cause explosion) and allowed to stand for 24 hours at 4 °C. The solution was dialyzed in 2 L of PBS with 20-30% of ethanol at 4 °C for two days. Two changes of buffer were made, PBS alone being used in the last one. The sample was centrifuged, quantified using Bradford method and stored at -20 °C.

Phase 1.2 Characterization of Dig-BSA conjugates

In order to estimate the number of digoxin molecules conjugated to BSA, absorbance at a wavelength of 388 nm of a digoxin solution in duplicate having known concentration was used as a reference. The reference and sample of Dig-BSA were completed with water up to 2 mL, then 10 mL of H2SO4 were added and the tubes were kept on standing at room temperature for 4 hours. Absorbance reading was taken and the number of moles of digoxin present in the Dig-BSA conjugate was calculated. The estimated number of digoxin molecules present in the conjugate was obtained from the ratio between the number of digoxin moles of Dig-BSA and the number of BSA moles added to obtain the conjugate.

Step 2) Obtaining a monoclonal anti-digoxin antibody

A monoclonal anti-digoxin antibody was generated by hybridoma technology through immunization of Balb/c mice with ovalbumin-digoxin conjugate in the Immunology Laboratory of the Heart Institute (Incor) of Hospital das Clinicas, School of Medicine, University of Sao Paulo (PAULA, 1993), under the guidance of Prof. Jorge Kalil.
Anti-digoxin hybridoma cells were grown in two 500 mL Spinner Flasks (Bellco Glass, Inc., USA), the first one containing 400 mL of medium and the other containing 300 mL of Dulbecco's Modified Eagle's Medium and Ham's F-12 Nutrient Mixture (DME/F12) (Sigma-Aldrich, Inc., USA) culture medium supplemented with 3% fetal bovine serum (Cultilab, Brazil) and 4 mM L-glutamine (Merck KGaA, Germany) in an oven (Forma Scientific Inc., USA) at 37 °C and 5% CO2. The viable cells counting was done with the aid of the exclusion dye Trypan blue and a Neubauer chamber. The cells were precipitated in a Centrifuge 5804 R (Eppendorf AG, Germany) at 5,000 x g for 10 minutes at 4 °C.

Phase 2.1 Purifying the monoclonal anti-digoxin antibody

Culture supernatants were purified by affinity chromatography in 245 mL of protein A-Sepharose 4FF resin (GE Healthcare) packed in a XK 50/30 column having 5 cm of diameter and 30 cm of height (GE Healthcare). The column was connected to a peristaltic pump P50 (GE Healthcare) that pumps the sample through the column; the column outlet was connected to a UV monitor at 280 nm UV1 (GE Healthcare), which was in turn connected to a REC 101 register (GE Healthcare). The balance of the column was performed by using a 1.5 M glycine/3 M sodium chloride buffer at pH 8.3; the column was subsequently loaded with the culture supernatant diluted in a 1:1 ratio in the balance buffer.

After loading, it was passed through the balance buffer column to remove any non-specific binding and to rebalance the column. A 50 mM sodium citrate/150 mM sodium chloride buffer at pH 4.3 was used for elution. The eluate was dialyzed twice against PBS overnight. After elution, the column was regenerated with a 50 mM glycine/150 mM sodium chloride buffer at pH 2.3 followed by a 50 mM Tris/1 M sodium chloride buffer at pH 8.6. Column rinsing was performed by passing a 50 mM Tris/150 mM sodium chloride at pH 8.6. During the loading step, a flow rate of 1 mL/min was used, and the remaining steps were performed at a flow rate of 10 mL/min.

Step 3) - Building the Fab library in pComb3X phagemid from anti-digoxin hybridomas

Phase 3.1 - Obtaining cDNA from anti-digoxin hybridomas

Anti-digoxin hybridoma cells were grown in a 250 ml Spinner Flask (Bellco Glass, Inc.) under the same conditions described in Step 2. The cultivation was precipitated at 5,000 x g for 10 minutes at 4 °C. The total RNA from hybridoma cells was extracted using the TRIZOL® Reagent (Invitrogen Corporation, USA) based on the method of isolation of RNA by phenol, guanidine isothiocyanate and chloroform (Chomczynski and Sacchi, 1987), according to manufacturer's instructions. The concentration of RNA was determined by spectrophotometry by absorbance at 260 nm (A260) in Ultrospec 1000 apparatus (Pharmacia Biotech (Biochrom) Ltd., England) and the equation used for calculation is: [RNA (pg/mL)] = 40 x A260 x dilution. The purity of the material was checked by the ratio of the absorbance at 260 nm and 280 nm (A260/A280). The optimum ratio A260/A280 for RNA varies from 1.9 to 2.0.

CDNA synthesis was performed by reverse transcription of total RNA using the kit SuperScript™ III First-Strand Synthesis System for RT-PCR (Invitrogem Corporation) using the Oligo (dT) 20 and following the manufacturer's instructions. The reactions were carried out in the thermocycler apparatus GeneAmp® PCR System 9700 (Applied Biosystems, USA).

Phase 3.2 Amplification of LC genes and portion Fd of HC

The cDNA of anti-digoxin hybridomas was used as a template to amplify the LC genes and Fd portion (VH and CH1) of HC using specific primer oligonucleotides for the kappa type (κ) of LC and IgG1 subclass (γ1) of HC of murines immunoglobulines, based on the Kabat database (Kabat et al. 1991).

Oligonucleotides containing specific restriction sites for cloning in the vector pComb3 (Barbas et al., 1991) were synthesized by Invitrogen Brasil Ltda. Six oligonucleotides 5' were used to the LC κ (κ1, κ2, κ3, κ4, κ5 and κ6) containing restriction site for the enzyme SacI and seven for the Fd portion (1A+1B, 2A, 2B, 2C, 3B+3C and 3D) with site for XhoI (ITOH et al. 1999) and the oligonucleotides 3' Mok3' and MoIgG1 containing restriction sites for XbaI and SpeI, respectively (Barbas et al. 2001).

The sequences of the oligonucleotides used are shown in Table 1.

Table 1 - Sequence of oligonucleotides used for amplification of the gene of the Fd portion of the heavy chain and light chain:

| Primer | Sequência |
|---|---|
| 1A+1B | SAGGTGCAGCTK**CTCGAG**TCAGGACCTRGC |
| 2A | SAGGTYCAGCTG**CTCGAG**TCTGGASCTGAG |
| 2B | CAGGTCCARCTG**CTCGAG**YCTGGGGCTGAG |
| 2C | GAGGTTCAGCTG**CTCGAG**TCTGKGGCWGAG |
| 3A | GARGTGAAGGTG**CTCGAG**TCTGGRGGAGGC |
| 3B+3C | GARGTGAAGCTT **CTCGAG**TCTGGAGGWGGC |
| 3D | GARGTGCAGCTG**CTCGAG**GGKGGGGGAGGA |
| k1 | GCGCGCGAGCTCGACRTTGTGATGWCACAGTCTCCATCCTYC |
| k2 | GCGCGCGAGCTCGATRTTKTGATGACCCARACTCCACTCTCC |
| k3 | GCGCGCGAGCTCGACATTGTGCTGACMCARTCTCCWGCTTC |
| k4 | GCGCGCGAGCTCSAAAWTGTKCTCACCCAGTCTCCAGCAATC |
| k5 | GCGCGCGAGCTCGAYATYCAGATGACMCAGWCTMCATCCTCC |

(continued)

| Primer | Sequência |
|---|---|
| k6 | GCGCGCGAGCTCCAAATTGTKCTCWCCCAGTCTCCAGCAATC |
| MoIgG1 | AGGCTTACTAGTACAATCCCTGGGCACAAT |
| MoK3' | GCGCCGTCTAGAATTAACACTCATTCCTGTTGAA |

Bold, underline, double underline and dotted represent, respectively, the sites of the restriction enzymes XhoI, SacI, SpeI and XbaI.

The LC and HC genes were amplified by the reaction of PCR using Taq DNA polymerase, recombinant - BR (Invitrogen Brazil Ltda.), in the apparatus GeneAmp® PCR System 9100 (Applied Biosystems, USA) starting at 94 °C for 2 minutes followed by 40 cycles of denaturation at 94 °C for 1 minute, annealing at 57 °C for 1 minute and elongation at 72 °C for 1 minute. A final elongation for 5 minutes at 72 °C was performed after the cycles (TSURUTA et al. 2003).

The DNA fragments amplified by PCR were analyzed by electrophoresis on agarose gel using Horizontal System of Electrophoresis LCH 7x8 (Loccus Biotechnology, Brazil). The 1.5% agarose gel was prepared with TAE buffer (40 mM Tris-EDTA, 5 mM sodium acetate, 1 mM EDTA, pH 8.0) and stained with SYBR® Safe DNA gel stain (Invitrogen Corporation). The samples were prepared with Blue/Orange Loading Dye, 6x (Promega Corporation, USA) and a marker of molecular weight 100bp DNA ladder (Promega Corporation) was used. The run was carried out for 40 minutes at 90 V. The result was visualized in blue light transilluminator device, Safe Imager™ Blue Light Transilluminator (Invitrogen Corporation) and images were captured by the system KODAK Gel Logic 100 Imaging System and the KODAK Molecular Image Software (Carestream Health, Inc., USA). After analysis, the amplification products of HC and LC genes were separately mixed, obtaining repertoires for constructing the combinatorial library.

The DNA concentration was determined by absorbance at 260 nm in a spectrophotometer Ultrospec 6300 Pro (Biochrom Ltd. England). Measurements were made on samples diluted in Milli-Q water, using quartz cuvettes of 10 mm. The used calculation was: [dsDNA ($\mu$g/mL)] = 50 x A260 x dilution. The purity of the material was checked by the A260/A280 ratio. An optimal ratio for the DNA varies from 1.8 to 1.9.

Phase 3.3 - Obtaining competent cells and vector

Sub-Phase 3.3.1 Preparation of cells of *Escherichia coli* XL1-Blue for transformation by heat shock

The bacterial strain of Escherichia coli used for cloning and expression of the recombinant antibody was XL1-Blue: recA1 EndA1 gyrA96 thi-1 hsdR17 supE44 relA1 lac [F' proAB lacIqZ1$\Delta$M15 Tn10 (Tetr)].

An aliquot of the bacteria *E. coli* Competent Cells XL1-Blue (Stratagene Corporation, USA) were plated on a plate of Luria-Bertani medium (LB) - agar (1% tryptone, 0.5% yeast extract, 1% NaCl, 1.5% agar; 0.1 M glucose) containing tetracycline at 10 $\mu$g/mL and incubated overnight at 37 °C. The next day, a colony was inoculated into 10 mL of Super broth medium (SB) (3% tryptone, yeast extract 2%, 1% MOPS, pH 7.0) containing tetracycline at 40 $\mu$g/mL and incubated overnight at 37 °C under stirring. From this culture 0.4 mL was taken and inoculated into 200 mL of SB containing 0.04 M glucose and 0.01 M MgCl2 and incubated at 37 °C under agitation until the absorbance at Ultrospec 1000 apparatus (Pharmacia Biotech (Biochrom) Ltd.) at 600 nm reached 0.8 - 0.9. The culture was incubated on ice for 20 minutes and centrifuged at 900 x g for 10 minutes at 4 °C (Centrifuge 5804 R - Eppendorf AG). The supernatant was discarded, the cells were resuspended in 50 mL of icy and sterile 0.1 M MgCl2 and incubated on ice for 20 minutes. The suspension was centrifuged at 900 x g for 10 minutes at 4 °C, the cells were resuspended in 25 mL of icy and sterile 0.1 M CaCl2 and incubated on ice for 20 minutes. A new centrifugation at 900 x g for 10 minutes at 4 °C was performed, the supernatant discarded and the cells resuspended in 2.4 mL of 0.1 M CaCl2 followed by the addition of 0.6 mL of glycerol. The suspension was divided into aliquots of 110 $\mu$L in icy tubes at ethanol bath with dry ice and stored at -80 °C. The title of the competent cells was determined by transforming thermal shock with a control DNA.

Sub-Phase 3.3.2 - The pComb3X vector

The pComb3XTT phagemid vector (Barbas et al., 2001) (Figure 4) was provided by Dr. Carlos Barbas at the Scripps Institute (The Scripps Research Institute, USA), along with the license for its use. It belongs to the family of pComb3X vectors {GenBank AF268281) and can be used to clone Fab fragments, scFv, peptides and proteins for the phage display system. This vector has the LC and HC genes of the human Fab fragment to the tetanus toxin - TT ‖, a single lacZ promoter, origin of replication F1 Ori and ampicillin resistance ampR. It contains two sequences of signal peptide, outer membrane protein (ompA) for LC and pectate lyase B (pe1B) for HC, which direct the polypeptide chains to the periplasm. The tail of 6 histidines (His6) and 10 hemagglutinines (HA) enable the purification and detection of the recombinant protein. It has an amber termination codon (TAG) which allows expression of soluble protein without the pIII in non-suppressor strains. For the expression of the soluble protein, it can also remove the protein pIII gene by digestion with the restriction enzymes SpeI and NheI.

Sub-Phase 3.3.3 - Transformation by heat shock

Amplification of the vector was made by heat shock transformation of the bacteria *E. coli* Competent Cells XL1-Blue (Stratagene Corporation) with pComb3XTT vector. Fifty microliters of the XL1-Blue bacteria were incubated with 0.5 μL of the pComb3XTT vector for 10 minutes on ice, 45 seconds at 42 °C in the apparatus Thermomixer compact (Eppendorf AG) and 5 min on ice. 450 μL of SOC medium was added (2% tryptone, 0.5% yeast extract, 0.05% NaCl, 20 mM KCl, 1 mM MgCl2, 20 mM glucose) and incubated for 1 hour with stirring at 37 °C. From this culture 200 μL were plated on a plate of LB-agar medium containing ampicillin (100 μg/mL) and incubated overnight at 37 °C.

Sub-Phase 3.3.4 - Plasmid Extraction

Plasmids were purified using the kit Maxiprep HiSpeed® Plasmid Maxi (Qiagen Sciences, Inc., USA) and Miniprep Wizard® Plus SV Minipreps DNA Purification System (Promega Corporation) according to the manufacturer's protocol.

3.3.5 Sub-Phase - Precipitation with ethanol

The ethanol precipitation was made by adding absolute ethanol (2.2 x the volume of the sample) and ammonium acetate (10% of sample volume) and incubated for 1 hour at -80 °C. After incubation, the samples were centrifuged at 20,800 x g for 15 minutes at 4 °C and sediments were washed twice with cold 70% ethanol and centrifuged at 20,800 x g for 5 minutes at 4 °C after each washing. The samples were resuspended in autoclaved Milli-Q water.

Substep 3.3.6 - DNA analysis by digestion with restriction enzymes

The purified DNA was digested for 2 hours at 37 °C with the restriction enzymes SacI and XbaI (New England Biolabs Inc., USA) to check the presence of the light chain and with the enzymes XhoI and SpeI and (New England Biolabs Inc.) to verify the insert of the heavy chain. Then, the DNAs were analyzed by electrophoresis on agarose gel. The molecular weight markers used were 1 kb DNA ladder (Promega Corporation) or 1 kb DNA Ladder (Invitrogen).

Sub-Phase 3.3.7 - Preparation of *Escherichia coli* electrocompetent cells XL1-Blue

An aliquot of the bacteria *E. coli* Competent Cells XL1-Blue (Stratagene Corporation) was plated on a plate of LB-agar medium containing tetracycline (10 μg/μL) and incubated overnight at 37 °C. The next day, a colony was inoculated into 10 ml of SB medium containing tetracycline (40 μg/mL) and incubated overnight at 37 °C under stirring. This 0.4 mL culture was taken and inoculated into 200 mL of SB containing 0.04 M glucose and 0.01 M MgCl2 and incubated at 37 °C under agitation until the OD at 600 nm reaches 08-09. The culture was incubated on ice for 20 minutes and centrifuged at 900 x g for 20 minutes at 4 °C. The supernatant was discarded and the cells were resuspended in 100 mL of sterile and icy 10% glycerol. The suspension was centrifuged at 1,500 x g for 20 minutes at 4 °C, resuspended in 50 mL of 10% glycerol and centrifuged at 2,500 x g for 30 minutes at 4 °C. The sediment was again resuspended in 50 mL of 10% glycerol and centrifuged at 3,000 x g for 30 minutes at 4 °C. The cells were then resuspended in 2 mL of 10% glycerol and divided in 150 icy tubes on dry ice bath / ethanol and stored at -80°C. The title was determined by electroporation with a DNA control.

Phase 3.4 - Cloning of the repertoire of LC genes in the vector pComb3X

The repertoire of LC genes cloned in the vector pComb3X for the construction of the LC library. Fifteen micrograms of the LC genes and the pComb3XTT vector were digested with the restriction enzymes SacI (10 U/μg) and XbaI (13 U/μg) at 37 °C overnight. The next day, the samples were precipitated with ethanol and resuspended in 20 μL of autoclaved Milli-Q water. The DNAs were applied in the agarose gel UltraPureAgarose® LMP Agarose - Low Melting Point (Invitrogen Corporation) and subjected to electrophoresis at 50 V for about 3 hours. As a standard, the marker 1 kb DNA Ladder was used (Promega Corporation). The bands with the approximate weight of 4,000 pb (vector) and of 700 pb (insert) were extracted from the gel with a scalpel and purified with the kit Wizard® SV Gel and PCR Clean-Up System (Promega Corporation), following manufacturer's instructions. The purified samples were precipitated with ethanol and resuspended in 10 μL of autoclaved Milli-Q water. The determination of the concentration of the DNA was performed using the apparatus QubitTM Fluoroweter (Invitrogen Corporation) and the kit Quant-iT dsDNA BR Assay kits (Invitrogen Corporation). Five hundred nanograms of LC genes were bound to 100 ng of vector pComb3X overnight at 23 °C using 2 U of Taq DNA ligase (Invitrogen Corporation).

Sub-Phase 3.4.1 Transformation by electroporation

Seventy microliters of *E. coli* electrocompetent XL1-blue were incubated with 2 μl of the above binding reaction in 0.4 cm electroporation cuvettes (Bio-Rad Laboratories, Inc., USA) for 10 minutes on ice. After incubation, the samples were given a pulse of 2500 V at 5.0 ms in the Multiporator® apparatus (Eppendorf AG). Immediately, there were added 3 mL of SOC medium and incubated under stirring for 1 hour at 37 °C. 200 L of the culture were plated on LB-agar plate containing ampicillin (100 μg/mL), and the plates were incubated overnight at 37 °C.

The library size was estimated by calculating the number of colonies in Colony Forming Unit (CFU). Colonies were also randomly chosen, inoculated in 5 mL of SB medium containing ampicillin (100 μg/mL) and incubated overnight at 37 °C under stirring. The next day, the inoculants were centrifuged and the plasmids were purified with the kit Wizard® Plus SV DNA Purification Minipreps System (Promega Corporation). Digestions were made using Restriction enzymes SacI and XbaI to verify the presence of the light chain.

The library of LC genes was amplified by electroporation transformation, and incubating 2 μl of the LC library with 70 μL of *E. coli* electrocompetent XL1-Blue. After electroporation and incubation for 1 hour at 37 °C, 20 mL of SB medium was added containing ampicillin (100 μg/mL) and incubated at 37 °C overnight. Plasmids were purified with the kit Wizard® Plus SV DNA Purification Minipreps System (Promega Corporation) and quantified by the absorbance at 260 nm in the spectrophotometer Ultrospec 6300 Pro (Biochrom Ltd.).

Phase.3.5 Cloning of the repertoire of HC genes in the LC library

For cloning of the genes of the Fd portion in the pComb3X vector containing the LC genes, the same materials and methods of cloning the repertoire of LC genes were used (Phase 3.4), with the exception of restriction enzymes. Ten micrograms of the repertoire of HC genes and of the library of LC genes contained in the pComb3X vector were digested with restriction enzymes SpeI {3 U/μg) and XhoI (6 U/μg) for 3 hours at 37 °C. In the binding reaction, there were used 300 ng of the HC genes, 100 ng of vector and 2 U of Taq DNA ligase (Invitrogen Corporation). The reaction took place overnight at 23 °C. 70 μL of the mixture were electroporated in *E. coli* XL1-Blue with 2 μL of the binding reaction in a Multiporator® apparatus (Eppendorf AG), 2.500 V, 5.0 ms, 0.4 cm cuvettes (Bio-Rad Laboratories, Inc.). After electroporation, there were added 3 ml of the SOC medium and incubated under stirring for 1 hour at 37 °C. 200 μl of the culture were plated on LB-agar plate containing ampicillin (100 g/μL) and incubated overnight at 37 °C.

The analysis of combinatorial library was made in a similar way as done for the LC library, adding digestions with restriction enzymes SpeI and XhoI to detect the presence of the Fd portion and the analysis of the diversity of combinatorial library, made by digestion with the BstNI restriction enzyme, which recognizes the CC/WGG site (New England Biolabs Inc.). The clones showing the inserts of LC and of HC were quantified by spectrophotometry and sequenced.

Sub-Phase 3.5.1 Sequencing and analysis of DNA

The DNA sequencing of the clones showing the two inserts was done using the primer oligonucleotide ompseq (5'-AAG ACA GCT ATC GCG ATT GCA G-3') for the light chain and the oligonucleotide pelseq (5'-ACC TAT TGC CTA CGG CAG CCG-3') for heavy chain (Barbas et al. 2001). The samples were prepared with BigDye® Terminator v3.1 Cycle Sequencing Kit (Applied Biosystems). The DNA Sequencing Service (SSDNA) of the Biochemistry Deparyment - IQUSP which performs the sequencing by the Sanger method was used, through the capillary sequencer ABI PRISM® 3100GeneticAnalyzer / HITACHI. Nucleotide sequences were analyzed using the Chromas lite program (Technelysium Pty Ltd.) and the amino acids were deduced using the translation program Expert Protein Analysis System (ExPASy), of the Swiss Institute of Bioinformatics (SIB). The sequences of the clones were compared to sequences of the GenBank using the program Basic Local Alignment Search Tool (BLAST) of the National Center for Biotechnology Information (NCBI), Bethesda, MD, USA (ALT3CHUL et al., 1990) and were aligned using the alignment program for DNA and proteins ClustalW2 of the European Bioinformatics Institute (EBI) belonging to the European Molecular Biology Laboratory (EMBL) (LARKIN et al., 2007). Identification of the CDRs was based on the Kabat numbering schema (1991).

Step 4) Phage display and enrichment of Fab library

The combinatorial library of anti-digoxin Fab fragments was enriched by phage display and panning with the immobilized antigen. A scheme showing the main steps of the library enrichment is shown in Figure 5.

Phase 4.1 Preparation of helper phage

An aliquot of the bacteria *E. coli* Competent Cells XL1-Blue (Stratagene) was plated on LB-agar plate containing tetracycline (10 μg/mL) and incubated overnight at 37 °C. A XL1-blue colony was inoculated into 10 mL of SB containing 10 μg/mL of tetracycline and incubated under stirring at 37 °C until the OD at 600 nm reaches 1.0. Then, there was added 1 plate of VCSM13 Interference-Resistant Helper Phage (Stratagene Corporation) and incubated for 2 hours at 37 °C. 2 ml of the infected culture were transferred to a 1 L Erlenmeyer flask containing 100 mL of SB with 10 μg/mL of tetracilcina and 70 μg/mL of kanamycin and incubated with stirring at 37 °C overnight (20 hours). The culture was centrifuged at 1,600 x g for 25 minutes at 4 °C and the supernatant was incubated in a water bath for 25 minutes at 70 °C followed by a further centrifugation at 1,600 x g for 25 minutes at 4 °C. The supernatants were stored at 4 °C (stable for months).

Sub-Step 4.1.1 Title determination of helper phage

There was done an inoculation of 10 μL of *E. coli* electrocompetent XL1-blue in 10 mL of SB with 10 μg/mL of tetracycline and incubated for 5-6 hours at 37 °C. Dilutions of $10^{-6}$, $10^{-7}$ and $10^{-8}$ of VCSM13 were prepared. 5μL of each dilution were added to 100 μL of XL1-Blue and incubated for 15 minutes at room temperature. 3 mL of soft agar was added (1% tryptone, 0.5% yeast extract, 0.5% NaCl, 0.65% agar) prewarmed (42 °C) and poured over the prewarmed LB-agar plate (37 °C). The plates were incubated overnight at 37 °C. Each formed lysis plate represents a viral particle suspension. The title of the phage was calculated as Plat Forming Units (PFU)/mL: n° of lysis plates /v (μL) phage x dilution factor x $10^3$ μL.

Sub-Phase 4.2 - Generation of the anti-digoxin Fab phages library (phage display)

Two microliters of the combinatorial library obtained above were transformed by electroporation into 70 μL of *E. coli* electrocompetent of XL1-Blue. After incubation for 1 hour at 37 °C, 50 μL of the culture were plated on LB-agar plate containing ampicillin (100 μg/mL) and incubated overnight at 37 °C to confirm the size of the combinatorial library. There was added to the culture 7 mL of SB containing ampicillin 100 μg/mL, tetracycline 10 μg/mL and glucose 40 nM. After incubation for 2 hours at 37 °C, there were added about 1 x $10^{11}$ PFU of helper phage, followed by further incubation for 2 hours at 37 °C. 0 culture was centrifuged at 2,200 x g for 10 minutes at 4 °C. The sediment was resuspended in 10 mL SB and centrifuged at 2,200 x g for 10 minutes at 4 °C. The sediment was again resuspended in 10 mL SB containing ampicillin 100 μg/mL and kanamycin 70 μg/mL and incubated overnight (20 hours) at 30 °C. The culture was centrifuged at 4,500 x g for 10 minutes at 4 °C and the supernatant was transferred to a new tube. To the supernatant there were added 2 mL of polyethylene glycol (PEG) 6,000 20%/2.5 M NaCl. The mixture was incubated for 30 minutes on ice and centrifuged at 4,500 x g for 30 minutes at 4 °C, the sediment was resuspended in 400 μL of 2% BSA/TBS and centrifuged at 20,800 x g for 5 minutes at 4 °C. 5 μL of the supernatant were separated to determine the title (dilution $10^{-8}$, $10^{-9}$) and the remainder was aliquoted and stored at 4 °C (phage library).

Sub-Phase 4.2.1 - Enrichment of anti-digoxin Fab phage library (panning).

A 96 well plate (Nunc, USA) were sensitized with 50 μL of the prepared previously Dig-BSA conjugate (see Phase 1.1) at 4 μg/mL. The plate was incubated at 4 °C overnight, followed by 3 washes with PBS. Blocking was done with 150 μl of 1% BSA/PBS and the plate was incubated for 1 hour at 37 °C. In each well 50 μL of the phage suspension were added, which were incubated for 2 hours at 37 °C and after incubation, the wells were washed 5x with 2% BSA/TBS. The elution was performed by incubation for 10 minutes at room temperature with 50 μL of elution buffer (0.1 M HCl - glycine, pH 2.2). Neutralization was done with 3 μL of 2 M Tris-base, and the contents of the wells were transferred to 1.5 mL tubes. An aliquot of 5 μL was separated to determine title (dilution $10^{-2}$, $10^{-4}$). A new cycle of panning was started by infecting 0.5 mL of *E. coli* XL1-Blue culture with 100 μL of eluted phages. After incubation for 15 minutes at room temperature, 100 μL were plated on LB-agar containing ampicillin (100 μg//mL) and incubated overnight at 37 °C while to the remainder, there were added 10 mL of SB, 100 μg/mL ampicillin, 10 μg/mL tetracycline and glucose 40 nM, continuing the panning procedure.

From the second cycle of panning, the efficiency of each cycle was calculated as a percentage of binding, as shown below:

Binding (%) = [(title of phages after panning) / (title of phages before panning)] x 100

The enrichment factor of the library was obtained by the ratio of the percentage of binding of the last cycle of panning with respect to the previous cycle.

After the third cycle of panning of combinatorial library, 10 clones were randomly selected from the dilution plate $10^{-4}$ to be analyzed as described in the combinatorial library analysis (item phase 3.5) and the clones showing the two inserts were sequenced as previously described. After analysis of the data sequencing, 4 clones were selected to express soluble Fab fragments.

Step 5) - Expression of soluble Fab fragments

The expression of soluble Fab fragments in bacteria *E. coli* of the XL1-Blue strain is possible after the removal of the gene III of the pComb3X vector by double digestion with restriction enzymes SpeI and NheI (New England Biolabs Inc.). After digestion with the enzymes for 2 hours at 37 °C, precipitation with ethanol was carried out. The samples were then subjected to electrophoresis on 1% agarose gel Agarose UltraPure® LMP - Low Melting Point (Invitrogen Corporation) 1%. Bands of approximately 4100 bp were isolated, purified with the kit Wizard® SV Gel and PCR Clean-Up System (Promega Corporation) and precipitated with ethanol. The vector lacking the gene III was rebound using 2 U of Taq DNA ligase (Invitrogen Corporation) for one night at 16 °C (~ 20 hours). To analyze the prepared vectors and confirm the withdrawal of the gene III, transformations were performed by electroporation on XL1-Blue. The plasmids were purified by Miniprep Wizard® Plus SV Minipreps DNA Purification System (Promega Corporation), precipitated with ethanol and analyzed by electrophoresis on a 1% agarose gel after digestion with the restriction enzyme XhoI and also by double digestion with restriction enzymes SacI and XbaI.

After the analyzes, 1 μL of each vector was transformed by heat shock at 50 μL of XL1-Blue for the expression of soluble Fab fragments. There were plated 20 μL and 100 μL on Petri dishes containing LB-agar with ampicillin (100 μg/mL) and the plates were incubated overnight at 37 °C. The next day, colonies were removed, inoculated into 10 mL of SB medium with ampicillin (100 μg/mL) and incubated for 2 hours at 37 °C. 2 ml of this culture were inoculated into a flask containing 100 mL of SB medium with ampicillin (100 μg/mL and 20 mM of MgCl2. Following, there was an incubation at 37 °C until the OD at 600 nm reaches approximately 1.0, when Isopropyl-β-D-Thiogalactoside (IPTG) was added at 0.5 mM. Culture was induced overnight (~ 16 hours) at 30 °C. After the induction, the culture wa s centrifuged at 2,200 xg at 4 °C for 15 minutes. The precipitate was resuspended in 5 ml of PBS and lysed by sonication (MICROSON™, Ultrasonic Cell Disruptor - Misonix Inc., USA): 10 pulses of 10 seconds interspersed with incubations of a 2 minute on ice. The suspension was centrifuged at 20,800 x g at 4 °C for 30 minutes. The supernatant was stored at -20 °C and used to confirm the binding to the antigen. A second induction was performed on a larger scale (1 L) to obtain Fab fragments for performing binding essays to the antigen, by inoculating 10 mL of the pre inoculation in 500 mL of SB medium with ampicillin (100 μg/mL) and 20 mm MgCl2 (2 vials).

Phase 5.1 - Quantification of the soluble Fab fragments by Enzyme-linked immunosorbent assay (ELISA) sandwich

The measurements were made in 96 wells MaxiSorp microplates (Nunc-Immuno™ Plates,). To each well, there were added 100 μL of sensitizing solution (pH 9.6 coating buffer: 0.16 g of 15 mM sodium carbonate (Na2CO3), 0.30 g of 35 mM sodium bicarbonate (NaHCO3), water qsp 100 mL), with the antibody AffiniPure F(ab')2 Fragment Rabbit-Anti-Mouse IgG, F(ab')2 Specific (Jackson ImmunoResearch Laboratories, Inc., USA) diluted at 1/400 (1.5 μg/mL), which were incubated in a humid chamber overnight at 4 °C. The following day, the plate was washed three times with PBS, each well was blocked with 300 μL of 1% BSA/PBS and the plate was incubated for 2 hours at 37 °C. After blocking, the plate was washed three times with PBS. The standard curve was prepared with ChromPure Mouse IgG Fab fragment (Jackson ImmunoResearch Laboratories, Inc.) at an initial concentration of 0.01 μg/mL and serial dilution 1/2 with 1% BSA/PBS. The crude extract containing Fab fragments was also diluted to the appropriate concentration in 1% BSA/PBS. The plate was incubated for 1 hour at 37 °C and then washed three times with PBS. There were added per well 100 μL of conjugated antibody Peroxidase-conjugated AffiniPure F(ab')2 Fragment Goat Anti-Mouse IgG, F(ab')2 Fragment Specific (Jackson ImmunoResearch Laboratories, Inc.) diluted in at 1/7.000 1% BSA/PBS and incubated for 1 hour at 37 °C. The plate was washed three times with PBS. There were added in each well 100 μL of the developing solution [10 mL of acetate buffer / 0.1 M citric acid pH 6.0 (1.36 g of sodium acetate in water qsp 100 mL, pH adjusted to 6.0 with 100 mM citric acid), 100 μL of 3,3',5,5'-tetramethylbenzidine (TMB) at 1% dimethylsulfoxide (DMSO);. 1,5 μL of hydrogen peroxide] and the plate was incubated for 20 minutes at room temperature, protected from light. The reaction was stopped with 50 μL of 4.7 N H2SO4 and then the absorbance at 450 nm was read using the plate reader (Labsystems iEMS Analyzer) and Genesis Lite software (Labsystems and Life Sciences International UK LTD.).

Step 6) - Characterization of crude extracts containing Fab fragments of clones obtained by phage display

Phase 6.1 - Binding assay of the soluble Fab fragment to the antigen by ELISA test.

The extracts of the four clones were used to analyze the binding of digoxin Fab fragments by the ELISA test as previously described (Phase 5.1) with some modifications. Sensitization was performed with Dig-BSA and BSA (negative control) at a concentration of 4 μg/mL. On an auxiliary plate, 260 μL of soluble Fab were placed in the 1st well of each column and the remaining were filled with 130 μL of 1% BSA/PBS. A serial dilution 1/2 was taken along the column. 100 mL from each well were transferred to the sensitized plate. The conjugated antibody used was Peroxidase-conjugated AffiniPure F(ab')2 Fragment Goat Anti-Mouse IgG, F(ab')2 Fragment Specific (Jackson ImmunoResearch Laboratories, Inc.) diluted 1/8.000. A similar assay was done with anti-digoxin monoclonal antibody produced by the hybridoma.

Phase 6.2 - Gel electrophoresis of polyacrylamide with sodium dodecyl sulphate (SDS-PAGE)

The electrophoreses were performed with polyacrylamide gels at concentrations ranging from 7.5% to 12%, with 0.75 mm or 1.0 mm thick depending on the experiment. The samples were diluted in PBS and prepared with sample buffer when necessary with or without reducing agent β-mercaptoethanol). The molecular weight markers used were the Low Molecular Weight Calibration Kit for SDS Electrophoresis (Amersham Biosciences UK Limited) and HMW-SDS Marker Kit (GE Healthcare).The samples were reduced by incubation for 5 minutes at 100 °C. The system used was a Mini-PROTEAN® Tetra Cell (Bio-Rad). The gels were stained with silver nitrate or Coomassie blue Dye R250.

Phase 6.3. - Assays for Western Blotting

Western blotting assays were always performed after SDS-PAGE of two identical gels. One gel was stained with silver nitrate or Coomassie, serving as a reference of the other, which was transferred to a membrane.

An assay test was done to detect Fab fragments expressed by the four clones. The crude extracts of the clones were applied in two 12% Polyacrylamide gels, which were subjected to electrophoresis. In one gel, Coomassie staining (reference) was performed, while the other was transferred to a polyvinylidene fluoride (PVDF) membrane (Millipore, USA) using the Trans-Blot SD Semi-Dry Electrophoretic Transfer Cell (Bio-Rad). The molecular weight marker used on the transferred gel was Kaleidoscope (Bio-Rad). The membrane was blocked with 10% skimmed milk/PBS, washed 3x with 0.1% Tween/PBS and incubated with the conjugated antibody Peroxidase-conjugated AffiniPure F(ab')2 Fragment Goat Anti-Mouse IgG, F(ab')2 Fragment Specific (Jackson ImmunoResearch Laboratories, Inc.) diluted 1/20.000 for 1 hour at 37 °C. The revelation was made with the kit Amersham ECL Plus Western Blotting Detection Reagents (GE Healthcare).

To analyze the binding of the Fab fragments by digoxin a test was made for each of the four clones, applying Dig-BSA and BSA on two 7.5% polyacrylamide gels, which were subjected to electrophoresis. In one gel, stained with silver nitrate (reference) was performed, while the other was transferred to a PVDF membrane as previously described. After blocking, each membrane was incubated with crude extracts of one of the clones for 1 hour at 37 °C. Immunodetection and development were carried out using the same reagents and conditions mentioned above.

Phase 6.4. - Analysis of affinity in the BIAcore® T100

The binding assay of Fab fragments present in the cell extracts by antigen was performed using the BIAcore® T100 (GE Healthcare) by surface plasmon resonance (SPR). The Dig-BSA conjugate, used as a binder, was diluted at acetate buffer pH 4.0 at a concentration of 50 μg/mL. Immobilization was taken on a sensor chip coated by carboxymethyl dextran, type CM5 (GE Healthcare) by amine coupling method (covalently bound with the dextran contained in the chip), with the target of 10,000 RU, using reactants from the covalent immobilization kit [1-ethyl-3-(3-dimethylamino-propyl)carbodiimide hydrochloride (EDC), N-hydroxysuccinimide (NHS), ethanolamine-HCl, pH 8.5] (GE Healthcare) and buffer HBS-EP (10 mM HEPES, 150 nm NaCl, 3 mM EDTA, 0.05% Tween 20 pH 7.4) as running buffer. Fab fragments of the crude extracts was quantified by ELISA (item 3.5.1), diluted in HBS-EP buffer at 2 kit g/mL and used as the analyte binding assay with the Dig-BSA conjugate immobilized on the chip. The assay was done with flow rate of 15 μL/min with a time of 40 s of contact of the analyte on the surface, and 40 s of dissociation. Regeneration was performed with 50 mM NaOH for 60 s at a flow rate of 30 μL/min.

The results of the method listed above and detailed can be defined as follows:

Result 1 - Obtainment and characterization of conjugated Dig-BSA

Digoxin was conjugated to bovine serum albumin (BSA) for use in combinatorial library enrichment of anti-digoxin Fab fragments by panning and in binding assays to the monoclonal antibody produced by anti-digoxin hybridoma and Fab fragments produced by the clones.
The digoxin number of residues for each BSA molecule was estimated to be 5.8 and was calculated as described on phase 1.2. The concentration of protein obtained by Bradford method was 10 mg/mL.
An ELISA test was done to verify the binding of the Dig-BSA conjugated to the anti-digoxin monoclonal antibody. The conjugate was immobilized at concentrations of 1, 2, 4 and 8 μg/mL in a microplate, and the culture supernatant of the anti-digoxin hybridoma was serially diluted with dilution factor 2 and applied as a primary antibody. The secondary antibody used was anti-IgG of mouse conjugated with peroxidase. Figure 6 shows the absorbances obtained at 450 nm as a function of dilution of hybridoma supernatant for each concentration of antigen. The monoclonal antibody showed the same binding profile of the Dig-BSA conjugate at the four concentrations of antigen.

Result 2 - Obtainment and purification of the anti-digoxin monoclonal antibody

The anti-digoxin monoclonal antibody present in the supernatant of the hybridoma culture was purified to be used as a reference of the binding assays to the conjugate. The cells of the anti-digoxin hybridoma were cultured in two flasks containing 400 mL and 300 mL of medium and at concentrations of 0.74 x $10^6$ cells/mL with 98% viability and 0.94 x $10^6$ cells/mL with 97% viability, respectively. After purification by affinity chromatography by protein A, the eluate was concentrated and analyzed by 12% polyacrylamide gel electrophoresis in the reduced form by β-ME and non reduced (Figure 7). A single band can be seen in the non reduced sample (channel 1), representing the whole antibody. In the reduced sample, the two characteristic bands can be seen representing the heavy and light chains of IgG. A non reduced weak band corresponding to the non reduced IgG can also be seen.
The purified anti-digoxin monoclonal antibody was also used to standardize the concentration of Dig-BSA immobilized in the ELISA tests. Concentrations of antigen of 0.5 to 10 μg/mL were immobilized, and purified IgG was used as primary antibody with an initial concentration of 500 μg/mL. The secondary antibody used was anti-IgG of mouse conjugated to peroxidase, diluted 1/10.000. In Figure 8, there is presented the profile of binding to anti-digoxin for 6 concentrations of Dig-BSA. The concentration of 4 μg/mL was chosen for the immobilization of Dig-BSA and of BSA in all ELISA tests.

Result 3 - Construction of the combinatorial library of anti-digoxin Fab fragments

Construction of the combinatorial library was started by obtaining the repertoires of LC and HC genes from the anti-digoxin hybridoma, followed by sequential cloning of these genes in the pComb3X vector.
Cells of the anti-digoxin hybridoma concentration of 0.95 x $10^6$ cells/mL with 96% viability were used for total RNA extraction from the cells. After extraction of total RNA, the obtained concentration was of 2.6 μg/mL and the A260/A280 ratio was 1.93. 0 cDNA synthesized by reverse transcription of mRNA was used to amplify the LC and HC genes, using starter oligonucleotides specific of genes for murine immunoglobulin of LC (κ) and Fd portion (VH and CH1) of HC (γ1). The DNA fragments amplified by PCR were analyzed by electrophoresis in 1.5% agarose gel (Figure 9). The amplification of the HC gene is shown in Figure 9A, where one can observe that the cDNA was not amplified using the oligonucleotide 1A + 1B (channel 1); the oligonucleotides 2A, 2B, 2C presented a band of approximately 650 bp (channels 2, 3 and 4) the oligonucleotides 3A, 3B + 3C presented a band of approximately 750 bp (channels 5 and 6) and the oligonucleotide 3D did not amplify (channel 7). In the amplification of the genes of LC (Figure 9B), it can be seen that there has been amplification using oligonucleotides k1 and k3, which presented bands of approximately 700 bp (channels 1 and 3) and κ6, which presented approximately 600 bp band (channel 6). After the analysis by electrophoresis, the DNA fragments of HC and LC were separately mixed, forming the repertoires of genes of LC and HC. These repertoires were sequentially cloned in the vector to construct the combinatorial library.
The LC repertoire of genes was the first to be cloned into the pComb3X vector for the construction of the LC library. The LC library was constructed by double digestion of the repertoire of LC genes and of the pComb3X vector with restriction enzymes SacI and XbaI, followed by electrophoresis on a 1% agarose gel for extraction and purification of the strips and binding thereof. The obtained library was transformed into *E. coli* XL1-blue. Its size was estimated at 3 x $10^5$ clones. 10 clones were randomly selected for DNA extraction and the presence of the LC insert was verified by electrophoresis on 1% agarose gel, after digestion with the restriction enzymes SacI and XbaI. From the 10 clones analyzed, only the clone 8 did not present a band of approximately 700 bp, corresponding to the insert LC (Figure 10). According to the samples which were tested, 90% of the clones from the LC library had the LC insert. The LC library was amplified for cloning of the repertoire of HC genes in this vector and obtaining combinatorial library of Fab fragments.
The LC library and the repertoire of HC genes were doubly digested with restriction enzymes SpeI and XhoI and subjected to electrophoresis. The bands were extracted, purified and bound to obtain the combinatorial library. The obtained library was size estimated at 5 x $10^6$ clones. 10 clones were selected at random to check the presence of LC and HC inserts by digestion with specific restriction enzymes. After agarose gel electrophoresis, it was found that from the 10 clones analyzed, 6 presented bands of approximately 700 bp relative to the HC inserts (Figure 11a) and LC (Figure 11B). The 10 selected clones were also digested with BstNI restriction enzyme to analyze the diversity of the obtained combinatorial library. Observing the 6 clones which presented the two inserts, it was not possible to note apparent differences in restriction pattern between clones 2, 3, 6, 8 and 10 (Figure 12). Clone 4 presented a different profile from the others.

The six clones that showed the LC and HC genes were sequenced. From the nucleotide sequences obtained from each clone, there were deduced sequences of amino acid residues and stop codons were detected in the genes of LC of all clones analyzed, indicating that none of the clones was functional, they were all pseudogenes. Clone 3 also had a stop codon in the gene of HC. Clones 2, 6, 8 and 10 presented identical amino acid sequences in the HC. Clone 4 had a glutamic acid residue (E) in the CDR2 region, while the others had a lysine (K) (data not shown). This library was used for further experiments.

Result 4 - Phage display and enhancement of anti-digoxin Fab library

The combinatorial library of anti-digoxin Fab fragments obtained by sequential cloning repertoires of genes of LC and HC was enriched after some rounds of phage display and panning, which selected the phages exposing anti-digoxin Fab fragments by binding to antigen Dig-BSA.

The phagemids of the library were transformed into *E. coli* cells XL1-Blue, which were infected by helper phage VCSM13. Phage showing antibodies were recovered from the culture and selected into rounds of panning with plate immobilized Dig-BSA.

The number of CFU before and after selection with antigen was calculated in each round in order to estimate the enrichment factor of the library. The results are shown in Table 2.

An increase of about 36 times in the enrichment factor can be noticed in the second round and only about 1 time in the third round, indicating that two rounds of panning were sufficient for the enrichment of anti-digoxin Fab library.

Table 2 - Titles of the phagemids before and after each round of panning and determining the efficiency of panning (% binding) and enrichment of the library

| Panning round | Phages before panning (CFU)[a] | Phages after panning (CFU)[b] | % Binding ($10^{-4}$)[C] | Enrichment[d] |
|---|---|---|---|---|
| 1 | 5.37 x $10^{12}$ | 4.13 x $10^6$ | 0.77 | - |
| 2 | 1.70 x $10^{11}$ | 4.72 x $10^6$ | 27.83 | 36.14 |
| 3 | 4.40 x $10^{11}$ | 1.10 x $10^7$ | 25 | 0.90 |
| a: number of CFU of phagemids incubated with Dig-BSA.<br>b: total number of CFU of phages contained in the eluates c: (phages after panning / Phages before panning) x 100.<br>d: enrichment of the library compared to the previous round of panning. | | | | |

After the third round of panning, 10 clones were randomly selected and the presence of inserts HC and LC was verified by digestion with restriction enzymes and analyzed by electrophoresis on 1% agarose gel. From the 10 clones analyzed, 6 presented the two inserts (Figure 13A and 13B). These 10 clones were analyzed by electrophoresis in 3% agarose gel after digestion with the restriction enzyme BstNI to verify library diversity. Observing the 6 clones that presented the two inserts, it was not possible to note apparent differences in the restriction pattern between clones 1, 2, 5, 9 and 10 (Figure 14). Clone 3 presented a different profile from the other clones.

Clones containing the LC and HC inserts (clones 1, 2, 3, 5, 9, 10) were analyzed by sequencing of HC and LC genes. The sequences of the deduced amino acid were aligned using the program ClustalW2. The numbering of the amino acids of the CDRs and the deduction was based on the scheme of Kabat et al. (1991). All clones had identical sequences amino acids in HC. Sequencing analysis of LC showed that clone 3 was a pseudogene and upon comparing its nucleotide sequence to sequences deposited in GenBank using the BLASTn program, found 100% identity to the gene Mus musculus immunoglobulin aberrantly rearranged kappa chain mRNA, partial sequence (GenBank: U56414.1). Clones 5 and 10 had identical sequences of amino acids of LC.

Clone 9 has a glutamine residue (Q) in position 54 of CDR2 region of the LC, while the other clones have an arginine (R). Clones 1, 2, 9 and 10 presented variations in the sequence of amino acids of framework1 region (Figure 15).

The deduced sequences of amino acid of the LC and HC of the clones 1, 2, 9 and 10 were examined for homology by BLASTp program, comparing to sequences deposited in GenBank. The sequences which presented higher identity with the clones are shown in Table 3 (LC) and Table 4 (HC), in percentage identity (number of identical amino acids / number of total amino acids of the sequences). Comparing the CDRs of LC, it was noted more than 77.8% identity in all the CDRs, wherein the CDR2 of the clones 1, 2 and 10 presented 100% identity with the three found sequences (Table 3), while CDRs of the clones of HC had lower percentages of identity with the found sequences, below 80%, reaching only 18.2% of identity in the CDR3 in one of the sequences (Table 4).

Table 3 - Analysis of homology of LC of the Fab fragments of the four anti-digoxin clones using BLASTp.

| IgM kappa LC MP-18-3-117 [Mus musculus](1) | | | | |
|---|---|---|---|---|
| | Identity | CDR 1 | CDR2 | CDR3 |
| Clone 1 | 96% (149/156) | | | |
| Clone 2 | 92% (144/157) | 90.9% (10/11) | 100% (7/7) | 88.9% (8/9) |
| Clone 10 | 92% (159/172) | | | |
| Clone 9 | 95% (164/173) | 90.9% (10/11) | 85.7% (6/7) | 88.9% (8/9) |
| LC AcMo agglutinator [Mus musculus](2) | | | | |
| | Identity | CDR 1 | CDR2 | CDR3 |
| Clone 1 | 96% (149/156) | | | |
| Clone 2 | 91% (145/160) | 81 8% (9/11) | 100% (7/7) | 77.8% (7/9) |
| Clone 10 | 92% (159/172) | | | |
| Clone 9 | 94% (165/175) | 81 8% (9/11) | 85.7% (6/7) | 77.8% (7/9) |
| kappa immunoglobulin anti-YGNNV [Mus musculus](3) | | | | |
| | Identity | CDR 1 | CDR2 | CDR3 |
| Clone 1 | 95% (148/56) | | | |
| Clone 2 | 91% (143/157) | 81 8% (9/11) | 100% | 88.9% (8/9) |
| Clone 10 | 92% (58/172) | | | |
| Clone 9 | 94% (63/173) | 81 8% (9/11) | 85.7% (6/7) | 88.9% (8/9) |

GenBank: AAG12167.1 (unpublished), (2) GenBank: CAA72328.1 (BONG et al, 1998.), (3) GenBank: AAN86781.1 (LAI et al, 2002)..

Table 4 - Analysis of homology HC of Fab fragments of the four anti-digoxin clones using BLASTp

| HC crystallographic structure II-18 Complexed Human to Antibody of Murine Reference 125-2h Fab(1) | | | |
|---|---|---|---|
| Identity | CDR1 | CDR2 | CDR3 |
| 86% (148/173) | 80% (8/10) | 58.8% (10/17) | 27.3% (3/11 |
| HC, Catalytic Antibody 4b2 in complex with its Hapten Amidino(2) | | | |
| Identity | CDR1 | CDR2 | CDR3 |
| 87% (150/174) | 70% (7/10) | 64.7% (1 1/17) | 18.2% (2/1) |
| HC, variable region of the immunoglobulin anti-TSHR [Mus musculus](3) | | | |
| Identity | CDR1 | CDR2 | CDR3 |
| 84% (144/173) | 50% (5/10) | 70.6% (12/17) | 36.4% (4/11) |
| (1) PDB: 2VXT_H (ARGIRIADI et al, 2009.); (2) PDB: 1F3D_H (GONÇALVES et al, 2000; GOLINELLI-PIMPANEAU et al, 2000); (3) GenBank: AAU816647.1 (COSTAGLIOLA et al, 2004).. | | | |

Results 5 - Expression and characterization of the binding of the anti-digoxin Fab fragments

Clones 1, 2, 9 and 10, which had distinct sequences of amino acids in the FR1 region of the LC were chosen to express soluble Fab using XL1-Blue strain of *E. coli.* Therefore, it was necessary to remove the gene III of the pComb3X vector of each clone by digestion with restriction enzymes SpeI and NheI, allowing the Fab fragments to be expressed in the soluble form without the pIII. The bands of the vectors (~ 4 kb) were separated from the band of the gene III (612 bp) by electrophoresis on a 1% agarose gel (Figure 16).

The plasmid DNA of each clone was purified, transformed into *E. coli* XL1-Blue and the expression of soluble Fab was induced by addition of IPTG. After induction, the bacteria were lysed by sonication, and crude extracts containing the Fab fragments from different clones were used in ELISA assays, Western blotting and SPR.

Initially, it was made the expression of Fab fragments in 100 mL of medium to verify binding of antibodies to digoxin by ELISA and the amount of the antibody expressed by each clone (data not shown). Then the expression of Fab fragments was performed in 1 L of medium to obtain the amount of antibody for performing the assays of binding to the antigen. The results presented herein refer to Fab fragments of the second expression.

An assay of Western blotting was performed to verify the expression of Fab fragments in crude extracts of the four clones. The samples not reduced of the crude extract of the clones were diluted 1/2, applied to 12% polyacrylamide gel and subjected to electrophoresis. The gel was transferred to a PVDF membrane, which was incubated with the anti-IgG antibody of mouse specific for F(ab')2 fragment conjugated to peroxidase. In all clones, there was recognized a band of approximately 50 kDa, corresponding to the size of the Fab fragment and also bands of 25 kDa, size correspondent to the the HC and LC of the Fab fragment, separately (Figure 17).

After the confirmation of the expression of Fab fragments in crude extracts, there was made a Western blotting to verify the binding of anti-digoxin antibody of the 4 clones to the antigen. The Dig-BSA conjugate and BSA negative control were applied to 7.5% polyacrylamide gels and subjected to electrophoresis. The gels were transferred to PVDF membranes, which were incubated with crude extracts of the four clones. The anti-digoxin antibody present in the 4 extracts recognized a band of approximately 67 kDa, corresponding to the Dig-BSA conjugate, but showed no binding to BSA, as expected (Figure 18).

The concentration of Fab fragments expressed by clones was determined by ELISA sandwich, immobilizing the anti-IgG antibody of specific mouse to F(ab')2 fragment in microplates. The Fab fragment of mouse was used to construct the standard curve. The concentrations of Fab fragments after the second induction were calculated from the standard curve, resulting in: clone 1 - 6.67 μg/mL; clone 2 - 10.08 μg/mL, clone 9 - 0.61 μg/mL and Clone 10 - 1.73 μg/mL. Clone 9 had the lowest yield among the clones.

The binding of Fab fragments from different clones to antigen was analyzed by ELISA. The Dig-BSA and BSA (control) conjugate was immobilized at 4 μg/mL in microplates, incubated with crude extracts containing Fab fragments of the clones, serially diluted 1/2 with initial concentration of 50 ng/mL, and incubated with anti-IgG antibody of specific mouse for F(ab')2 fragment conjugated to peroxidase. The absorbance values were graphically plotted as a function of the titration of the concentrations of Fab fragments of the clones. The results show specific binding of the four clones to Dig-BSA, without evidence of binding to BSA (Figure 19).

From the results of the immunoassays, no biding difference was found between the four Fab fragments when analyzed in relation to the antigen. The ranking was made possible by the SPR analysis in the BIAcore®, a system that has a high sensitivity for the analysis of biomolecular interactions in real time. This system measures the angle change of the refractive index according to the mass of the molecule present on the surface of a chip.

Before performing binding assay, it was verified the absence of unspecific binding of the Fab fragments to the dextran contained in the surface of the chip. After the above test, the Dig-BSA conjugate was immobilized on the chip with the target of 10,000 RU and had as the final result the density of the immobilized protein of 9259.8 RU. The crude extracts of the four clones containing the Fab fragments at the concentration of 2.0 μg/mL were applied to the chip channels interacting with the Dig-BSA and generating a response measured in resonance units (RU). The RUs measured 5 seconds after the conclusion of the application of the fragments in the immobilized chip with Dig-BSA were: clone 1 - 495.9 RU; clone 2 - 476.3 RU; clone 9 - 206.9 RU; clone 10 - 1,023.5 RU (Figure 20). The clone 9, which has in its sequence of deduced amino acids a different amino acid residue in the CDR2 region of LC compared to the other clones, presented the lowest response among the analyzed clones. The four clones differ from each other in the sequences of amino acids in the FR1 region of the LC.

It was proved that the method allowed to obtain Fab fragments of anti-digoxin monoclonal antibody by use of phage display technology. After enrichment of the phages library, 10 clones were randomly selected and from these, 6 presented the LC and HC inserts and had regions of LC and HC genes analyzed by sequencing. Four clones presented differences in amino acid sequence of the LC, all in the region of the framework 1. These clones were expressed and presented binding to the antigen Dig-BSA in ELISA assays, Western blotting and SPR. None of the clones presented binding to BSA alone.

The phage display technology has brought many advantages, such as the selection of monoclonal antibodies from the library of variable human genes (Marks et al., 1991) and identification of therapeutic markers - *in vivo* (ARAP, 2005). Fab fragments can be expressed in *Escherichia coli* in large quantities and the recombinant DNA technology provides the opportunity for the introduction of mutations that can optimize Fab, increasing the expressed amount, stability, solubility and facilitating the humanization and affinity maturation (KWONG, RADER, 2009).

The quantity of Fab obtained per liter of culture in *E. coli* depends primarily on the amino acid sequence of Fab (KWONG, RADER, 2009) and thus may vary among clones.

For the expression of soluble proteins in *E. coli* XL1-Blue using the pComb3X vector, it was necessary to remove the gene III vector and with it, the histidine tail which would allow purification by chromatography of chelated metals was lost.

Despite this disadvantage, this strategy was chosen to allow obtaining the isolated Fab fragment without the need for removal of the histidine tail after expression. Thus, the purification method should take this aspect into account. Conventional methods as ELISA and Western blotting confirmed the specificity of binding of Fab fragments to Dig-BSA antigen, but were not able to discriminate differences between the bindings of the four clones.

A more powerful and sensitive technique such as SPR allowed to show differences and classify the clones. BIAcore® is a system that has a high sensitivity for the analysis of biomolecular interactions in real time. SPR measures the change of the angle of the refractive index as the mass of this molecule. The detection occurs on the surface of a chip, wherein a binder is immobilized. The analyte passes through the chip channels, interacting with the binder and generating a response, measured in resonance units (RU) (SCHASFOORT, TUDOS, 2008). The relative response cannot be measured during the application of the analyte, as there may be a change in the refractive index caused by the crude extract on the surface (bulk effect), and not a specific interaction between binder and analyte. This difference is nullified when the relative response is measured after 5 seconds from the end of the injection of the analyte. A flow cell containing immobilized BSA was used as a reference for analysis of binding of the anti-digoxin clones, with negative result. The assay indicated differences in the binding between anti-digoxin clones and the antigen, with results ranging from 206.9 RU to 1023.5 RU. The clone 9, which has a glutamine (Q) substituting an arginine (R) at position 54 in the CDR2 region of the LC showed the lowest binding between the analyzed clones. The clone 10 showed the highest binding affinity, whereas clones 1 and 2 presented a similar and intermediate affinity to each other. Clones obtained by phage display have binding affinity greater than that obtained by the original antibody produced by the murine hybridoma which originated the work.

It is believed, therefore, that the subject invention allows the generation of monoclonal anti-digoxin Fab fragments with greater affinity and specificity than polyclonals provided the serum of animals. The phage display technology was used to achieve this goal. Monoclonal Fab fragments obtained with this method represent a potential of a product with specific power and more precise dose to detoxification of patients under digoxin therapy.

REFERENCES

ABBAS, A. K.; LICHTMAN, A. H. Imunologia celular e molecular, 5. ed. Rio de Janeiro: Elsevier, 2005. 576 p.

AGGARWAL, S. What's fueling the biotech engine-2009-2010. Nat. Biotechnol., v. 28, p. 1165-1171, 2010.

ALTSCHUL, S. F.; GISH, W.; MILLER, W.; MYERS, E. W.; LIPMAN, D. J. Basic local alignment search tool. J. Mol. Biol., v. 215, p. 403-410, 1990.

ANDRÉS, V. L. G. Revisión sistemática sobre la efectividad e indicaciones de los anticuerpos antidigoxina en la intoxicación digitálica. Rev Esp Cardiol., v. 53, p. 49-58, 2000.

ANT AN, E. M.; SMITH, T. W. Digitalis toxicity. Annu. Rev. Med., v. 36, p. 357-367, 1985.

ANTMAN, E. M.; WENGER, T. L.; BUTLER, V. P. JR.; HABER, E.; SMITH, T. W. Treatment of 150 cases of life-threatening digitalis intoxication with digoxin-specific Fab antibody fragments. Final report of a multicenter study. Circulation, v. 81, p.1744-1752, 1990.

ARAP, M. A. Phage display technology - Applications and innovations. Genet. Mol. Biol., v. 28, p. 1-9,2005.

ARGIRIADI, M. A.; XIA G, T.; WU, C; GHAYUR, T.; BORHANI, D. W. Unusual water-mediated antigenic recognition of the proinflammatory cytokine interleukin-18. J. Biol. Chem., v. 36, p. 24478-4489, 2009.

BARBAS, C. F. 3RD.; KANG, A. S.; LERNER, R. A.; BENKOVIC, S. J. Assembly of combinatorial antibody libraries on phage surfaces: the gene III site. Proc. Natl. Acad. Sci. U. S. A., v. 88 p. 7978-7982, 1991.

BARBAS, C. F.; BURTON, D. R.; SCOTT, J. K. SILVERMAN, G. J. Phage display: a laboratory manual. New York: Cold Spring Harbor Laboratory Press, 2001. 736 p.

BONG, Y. S.; CHO, S. H.; NHAM, S. U.; LEE, Y. I. Cloning and characterization of cDNAs coding for heavy and light chains of agglutinating monoclonal antibody (HAG12is1rh) specific for human red blood cells. Biochim. Biophys. Acta, v. 10, p. 156-158, 1998.

BUTLER, JR, V. P.; CHEN, J. P. Digoxin-specific antibodies.Proc. Natl. Acad. Sci. U. S. A., v. 57, p. 71-78, 1967.

BUTLER, JR, V. P.; SCHMIDT, D. H.; SMITH, T. W.; HABER, E.;RAYNOR, B. D.; DEMARTINI, P. Effects of sheep digoxin- specific antibodies and their Fab fragments on digoxin pharmacokinetics in dogs. J. Clin. Invest., v. 59, p. 345-359, 1977.

CALDAS, C; COELHO, V; KALIL, J.; MORO, A. M.; MARANHAO, A. Q.; BRÍGIDO, M. M. Humanization of the anti-CD18 antibody 6.7: an unexpected effect of a framework residue in binding to antigen. Mol. Immunol., v. 39, p. 941-952, 2003.

CHOMCZYNSKI, P.; SACCHI, N. Single-step method of RNA isolation by acid guanidinium thiocyanate-phenol-chloroform extraction. Anal. Biochem., v.162, p. 156-159, 1987.

CLACKSON, T.; HOOGENBOOM, H. R.; GRIFFITHS, A. D.; WINTER, G. Making antibody fragments using phage display libraries. Nature, v. 352, p. 624-628, 1991.

COSTAGLIOLA S, BONOMI M, MORGENTHALER NG, VAN DURME J, PANNEELS V, REFETOFF S, VASSART G. Delineation of the discontinuous-conformational epitope of a monoclonal antibody displaying full in vitro and in vivo thyrotropin activity. Mol Endocrinol., v. 18, p. 3020-3034, 2004.

EICHHORN, E. J.; GHEORGHIADE, M. Digoxin. Prog. Cardiovasc. Dis., v. 44, p. 251-266, 2002. ERLANGER, B. F.; BEISER, S. M. Antibodies specific for ribonucleosides and ribonucleotides and their reaction with DNA. Proc. Natl. Acad. Sci. U. S. A., v. 52, p. 68-74, 1964.

FLANAGAN, R. J.; JONES, A. L. Fab antibody fragments: some applications in clinical toxicology. Drug Saf., v. 27, p.1115-1133, 2004.

GHEORGHIADE, M.; VAN VELDHUISEN, D. J.; COLUCCI, W. S. Contemporary use of digoxin in the management of cardiovascular disorders. Circulation, v. 113, p. 2556- 2564, 2006.

GOLINELLI-PIMPANEAU, B.; GONCALVES, O.; DINTINGER, T.; BLANCHARD, D.; KNOSSOW, M.; TELLIER, C. Structural evidence for a programmed general base in the active site of a catalytic antibody. Proc. Natl. Acad. Sci. U. S. A., v. 18, p. 9892-9895, 2000.

GONÇALVES, O.;. DINTINGER, T.; LEBRETON, J.; BLANCHARD, D.; TELLIER, C. Mechanism of an antibody-catalysed allylic isomerization. Biochem. J., v. 3, p. 691-698, 2000. HORWITZ AH, NADELL R, PREUGSCHAT F, BETTER M. Chimeric immunoglobulin light chains are secreted at different levels: influence of framework-1 amino acids. Mol. Immunol., v. 31, p. 683-692, 1994. HUNTER, M. M.; MARGOLIES, M. N.; JU, A.; HABER, E. High-affinity monoclonal antibodies to the cardiac glycoside, digoxin. J. Immunol., v. 129, p. 1165-1172, 1982.

HUSE, W. D.; SASTRY, L.; IVERSON, S. A.; KANG, A. S.; ALTING-MEES, M.; BURTON, D. R.; BENKOVIC, S. J.; LERNER, R. A. Generation of a large combinatorial library of the immunoglobulin repertoire in phage lambda. Science, v. 246, p. 1275-1281, 1989.

KABAT, E. A.; WU, T. T.; PERRY, H. M.; GOTTES AN, K. S.; FOELLER, C. Sequences of proteins of immunological interest, 5. ed. Bethesda, MD: National Institute of Health, 2001. 2597 p.

KOHLER, G.; MILSTEIN, C. Continuous cultures of fused cells secreting antibody of predefined specificity. Nature, v. 256 p.495-497, 1975.

KRYKBAEV, R. A.; LIU, W. R.; JEFFREY, P. D.; MARGOLIES, M. N. (2001) Phage display-selected sequences of the heavy- chain CDR3 loop of the anti-digoxin antibody 26-10 define a high affinity binding site for position 16-substituted analogs of digoxin. J. Biol. Chem., v. 276, p. 8149-8158, 2001.

KRYKBAEV, R. A.; TSANTILI, P.; JEFFREY, P. D.; MARGOLIES, M. N. Modifying specificity of antidigoxin antibodies using insertional mutagenesis. Protein Sci., v. 11, p. 2899-2908, 2002.

KUBA, H.; FURUKA A, A.; OKAJIMA, T.; FURUKAWA, K. Efficient bacterial production of functional antibody fragments using a phagemid vector. Protein Expr. Purif., v. 58, p. 292-300, 2008.

KWONG, K. Y.; RADER, C. E. coli expression and purification of Fab antibody fragments. Curr. Protoc. Protein Sci., v. 55:, p. 6.10.1-6.10.14, 2009. Cap.6

LAI, Y. S.; JOHN, J. A.; GUO, I. C.; CHEN, S. C.; FANG, K.; CHANG, C. Y. In vitro efficiency of intra- and extracellular immunization with mouse anti-YGNNV antibody against yellow grouper nervous necrosis virus. Vaccine, v. 20, p. 3221-3229, 2002.

LAPOSTOLLE, F.; BORRON, S. W.; VERDIE, C.; TABOULET, P.; GUERRIER, G.; ADNET, F.; CLE ESSY, J. L.; BISMUTH, C.; BAUD, F. J. Digoxin-specific Fab fragments as single first- line therapy in digitalis poisoning. Crit. Care Med., v. 36, p. 3014-3018, 2008.

LARKIN, M. A.; BLACKSHIELDS, G.; BROWN, N. P.; CHENNA, R.; CGETTIGAN, P. A.; MCWILLIAM, H.; VALENTIN, F.; WALLACE, I. M.; WILM, A.; LOPEZ, R.; THOMPSON, J. D.; GIBSON, T. J.; HIGGINS, D. G. ClustalW and ClustalX version 2.0. Bioinfomatics, v.23, p. 2947-2948, 2007.

LEVY, R.; MOLINEUX, I. J.; IVERSON, B. L.; GEORGIOU, G. Isolation of trans-acting genes that enhance soluble expression of scFv antibodies in the E. coli cytoplasm by lambda phage display. J Immunol. Meth., v. 321, p. 164-173, 2007.

MARKS, J. D. HOOGENBOOM, H. R.; BONNERT, T. P.; MCCAFFERTY, J.; GRIFFITHS, A. D.; WINTER, G. By-passing immunization. Human antibodies from V-gene libraries displayed on phage. J. Mol. Biol., v. 222, p.581-597, 1991.

MCCAFFERTY, J.; GRIFFITHS, A. D.; WINTER, G.; CHISWELL, D. J. Phage antibodies: filamentous phage displaying antibody variable domains. Nature, v. 348, p. 552-554, 1990.

MORO, A. M.; RODRIGUES, M. T. A. Anticorpos monoclonais para a clinica. Biotecnologia Ciência e Desenvolvimento, v. 22, p. 32-35, 2001.

MUDGETT-HUNTER, M.; ANDERSON, W.; HABER, E.; MARGOLIES, M. N. Binding and structural diversity among high-affinity monoclonal anti-digoxin antibodies. Mol. Immunol., v. 22, p. 477-488, 1985. ORLANDI, R.; GUSSOW, D. H.; JONES, P. T.; WINTER, G. Cloning immunoglobulin variable domains for expression by the polymerase chain reaction. Proc. Natl. Acad. Sci. U. S. A., v. 86, p. 3833-3837, 1989.

PANKA, D. J.; MUDGETT-HUNTER,.; PARKS, D. R.; PETERSON, L. L.; HERZENBERG, L. A.; HABER, E.; MARGOLIES, M. N. Variable region framework differences result in decreased or increased affinity of variant anti-digoxin antibodies. Proc. Natl. Acad. Sci. U. S. A., v. 85, p. 3080-3084, 1988.

PAULA, F. J. DE. Fração sérica de pacientes urêmicos expandidos com atividade digoxina-símile inibidora da Na+K+ ATPase: isolamento, efeitos biológicos e caracterização com anticorpos monoclonais. 152 f. Tese (Doutorado em Nefrologia) - Faculdade de Medicina, Universidade de São Paulo, São Paulo, 1993. RINDERKNECHT, M.; VILLA, A.; BALLMER-HOFER, K.; NERI, D.; DETMAR, M. Phage-derived fully human monoclonal antibody fragments to human vascular endothelial growth factor-C block its interaction with VEGF receptor-2 and 3. PLoS One., v. 5, p. e11941, 2010.

SCHASFOORT, R. B. M.; TUDOS, A. J. Handbook of surface plasmon resonance. Cambridge: Royal Society of Chemistry Publishing, 2008. 403 p.

SCHILDBACH, J. F,; PANKA, D. J.; PARKS, D. R.; JAGER, G. C; NOVOTNY, J.; HERZENBERG, L. A.; MUDGETT-HUNTER, M.; BRUCCOLERI, R. E.; HABER, E.; MARGOLIES, M. N. Altered hapten recognition by two anti-digoxin hybridoma variants due to variable region point mutations. J. Biol. Chem., v. 266, p. 4640-4647, 1991.

SCHILDBACH, J. F.; NEAR, R. I.; BRUCCOLERI, R. E.; HABER, E.; JEFFREY, P. D.; NG, S. C.; NOVOTNY, J.; SHERIFF, S.; MARGOLIES, M. N. Heavy chain position 50 is a determinant of affinity and specificity for the anti-digoxin antibody 26-10. J. Biol. Chem., v. 268, p. 21739-21747, 1993.

SHORT, M. K.; JEFFREY, P. D.; KWONG, R. F; MARGOLIES, M. N. Contribution of antibody heavy chain CDR1 to digoxin binding analyzed by random mutagenesis of phage-displayed Fab 26-10. J. Biol. Chem., v. 270, p. 28541-28550, 1995. SHORT, M. K.; KRYKBAEV, R. A.; JEFFREY, P. D.; MARGOLIES, M. N. Complementary combining site contact residue mutations of the anti-digoxin Fab 26-10 permit high affinity wild-type binding. J. Biol. Chem., v. 277, p. 16365-16370, 2002.

SMITH, G. P. Filamentous fusion phage: novel expression vectors that display cloned antigens on the virion surface. Science, v. 228, p. 1315-1317, 1985.

SMITH, T. W.; BUTLER, V. P. JR.; HABER, E. Characterization of antibodies of high affinity and specificity for the digitalis glycoside digoxin. Biochemistry, v. 9, p. 331- 337, 1970.

SMITH, T. W.; HABER, E.; YETMAN, L.; BUTLER, JR. Reversal of advanced digoxin intoxication with Fab fragments of digoxin-specific antibodies. N. Eng J. Med., v. 294, p. 797-800, 1976.

STRACHAN, G.; MCELHINEY, J.; DREVER, MR.; MCINTOSH, F.; LAWTO, L. A.; PORTER, A. J. Rapid selection of anti-hapten antibodies isolated from synthetic and semi-synthetic antibody phage display libraries expressed in Escherichia coli. FEMS Microbiol Lett., v. 210, p. 257-261, 2002.

TONEGAWA, S. Somatic generation of antibody diversity. Nature, v. 302, p. 575-581, 1983.

TSURUTA, L. R.; TOMIOKA, Y.; HISHINUMA, T.; KATO, Y.; ITOH, K.; SUZUKI, T.; GGURI, H.; HIRAMA,.; GOTO, J.; MIZUGAKI, M. Characterization of 11-dehydro-thromboxane B2 recombinant antibody obtained by phage display technology. Prostaglandins Leukot Essent Fatty Acids, v. 68, p. 273- 284, 2003.

WITHERING, W. An account of the foxglove and some of its medicai uses; with practical remarks on the dropsy, and some other diseases In: Willins FA, Keys TE, eds. Classics of Cardiology, New York, NY: Henry Schyuman, Dover Publications; 1941; cap. 1, p. 231-252.

## Claims

**1.** METHOD FOR PRODUCING AND OBTAINING VARIABLE DOMAINS OF ANTI-DIGOXIN ANTIBODY FAB FRAGMENT USING THE MOLECULAR BIOLOGY CLONING TECHNIQUE **characterized by** the fact that the method for producing and obtaining clones of anti-digoxin antibody Fab fragment using cloning technology in molecular biology by phage display and the characterization of its antigen binding, comprises the following steps:

Step 1) - Obtaining and characterizing bovine albumin conjugated with digoxin (Dig-BSA)

Phase 1.1 - Obtaining Dig-BSA conjugates;
Phase 1.2 - Characterizing Dig-BSA conjugates;

Step 2) - Preparing the anti-digoxin monoclonal antibody; Phase 2.1 - Purifying the monoclonal anti-digoxin antibody;
Step 3) - Building the Fab library in pComb3X phagemid from anti-digoxin hybridomas

Phase 3.1 - Obtaining cDNA from anti-digoxin hybridomas; Phase 3.2 - Amplifying genes of LC and of Fd portion of HC;
Phase 3.3 - Obtaining competent cells and vector;
Phase 3.4 - Cloning the LC repertoire of genes in vector pComb3X;
Phase 3.5 - Cloning the HC repertoire of genes in the LC library;

Step 4) - Phage display and enrichment of Fab library;

Phase 4.1 - Preparing helper phage;
Phase 4.2 - Generating anti-digoxin Fab phage library (phage display);

Step 5) - Expressing soluble Fab fragments;

Phase 5.1 - Quantifying soluble Fab fragments by sandwich enzyme-linked immunosorbent assay (ELISA);

Step 6) - Characterizing crude extracts containing Fab fragments of clones obtained by phage display

Phase 6.1 - Soluble Fab fragment antigen binding assay by ELISA test;
Stage 6.2 - Polyacrylamide gel electrophoresis with sodium dodecyl sulphate (SDS-PAGE);
Phase 6.3 - Western blotting assay;
Phase 6.4 - Affinity analysis using BIAcore T100.

**2.** The METHOD according to claim 1, **characterized** by extracting total RNA from anti-digoxin hybridomas and amplifying LC and HC genes, Fd portion (VH and CH1) of immunoglobulins by PCR.

**3.** The METHOD according to claim 1, **characterized** by enrichment of the combinatorial library of anti-digoxin Fab fragments by panning and in binding assays to monoclonal antibody produced by anti-digoxin hybridoma and to Fab fragments produced by the clones comprising that digoxin is conjugated to bovine serum albumin (BSA).

**4.** The METHOD according to claim 1, **characterized** by selecting anti-digoxin clones by binding to antigen.

**5.** The METHOD according to claim 1, **characterized** by expressing soluble anti-digoxin Fab fragments.

**6.** The METHOD according to the previous claims, **characterized** by concentrations of Fab fragments resulting in: clone 1- 6.67 g/mL, clone 2 - 10.08 μg/mL, clone 9 - 0.61 μg/mL and clone 10 - 1.73 μg/mL.

**7.** The METHOD according to the previous claims, **characterized** by occuring specific binding of the four clones to Dig-BSA without evidence of linkage to BSA.

FIG.1

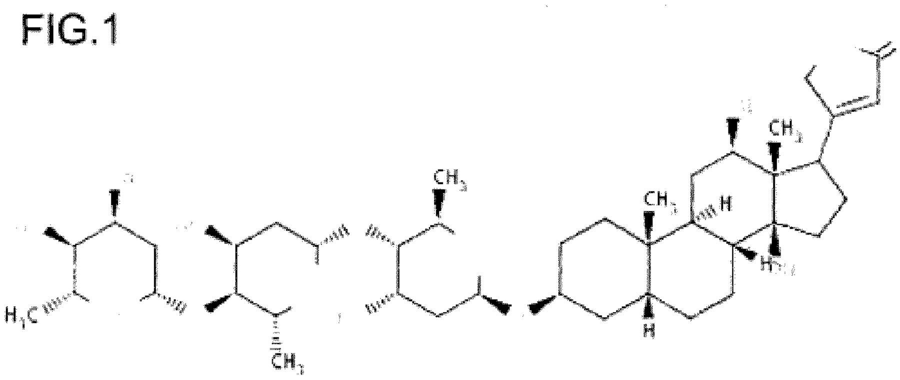

FIG.2

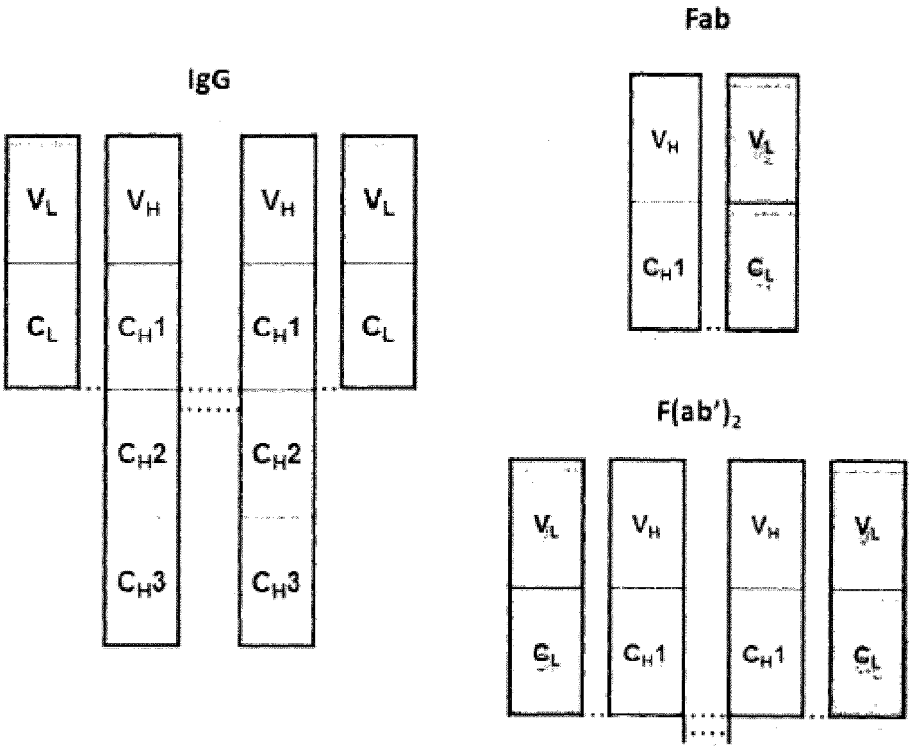
IgG
$V_L$
$V_H$
$V_H$
$V_L$
$C_L$
$C_H1$
$C_H1$
$C_L$
$C_H2$
$C_H2$
$C_H3$
$C_H3$
Fab
$V_H$
$V_L$
$C_H1$
$C_L$
$F(ab')_2$
$V_L$
$V_H$
$V_H$
$V_L$
$C_L$
$C_H1$
$C_H1$
$C_L$

FIG.3

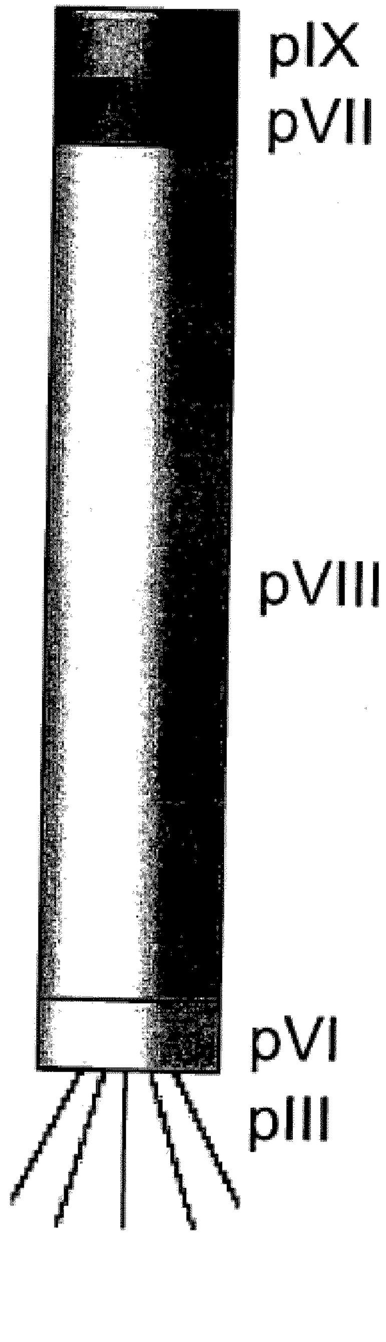

FIG.4

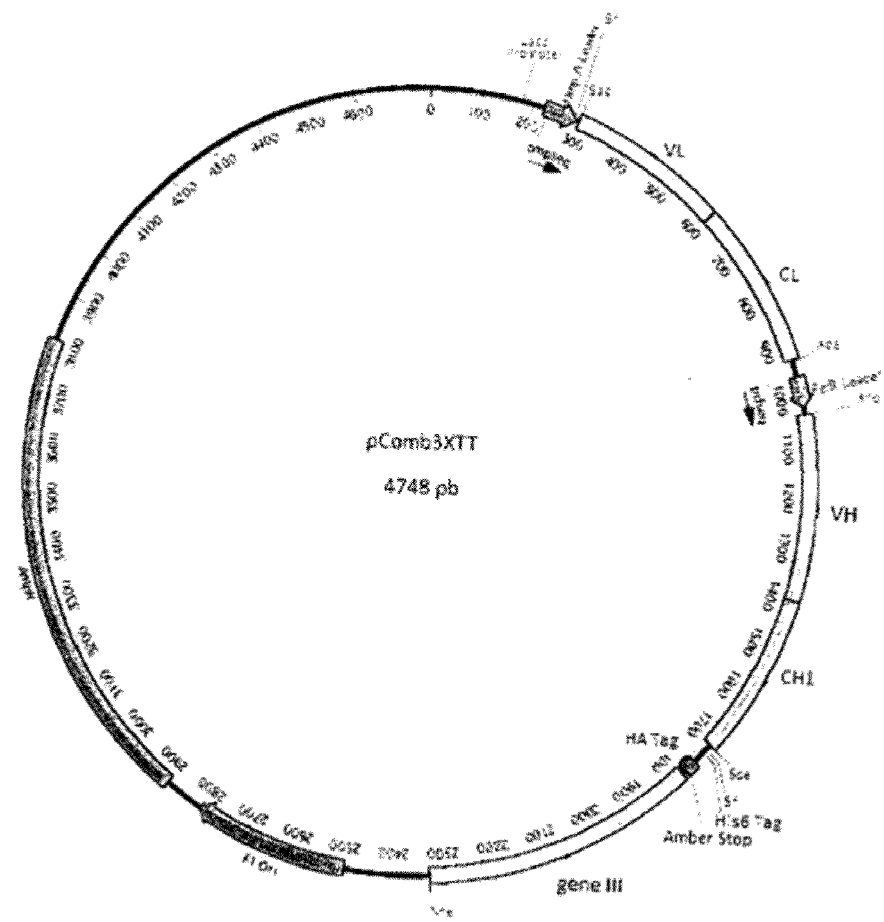
pComb3XTT
4748 pb
VL
CL
VH
CH1
HA Tag
His6 Tag
Amber Stop
gene III

FIG.5

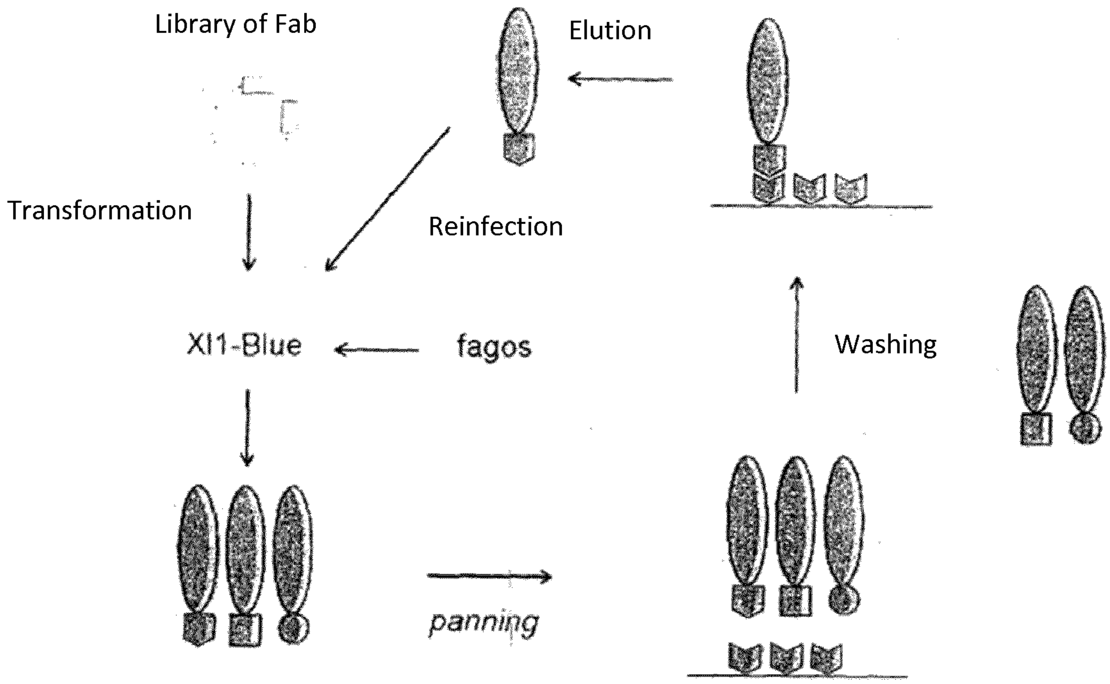
Library of Fab
Elution
Transformation
Reinfection
Xl1-Blue
fagos
Washing
panning

FIG.6

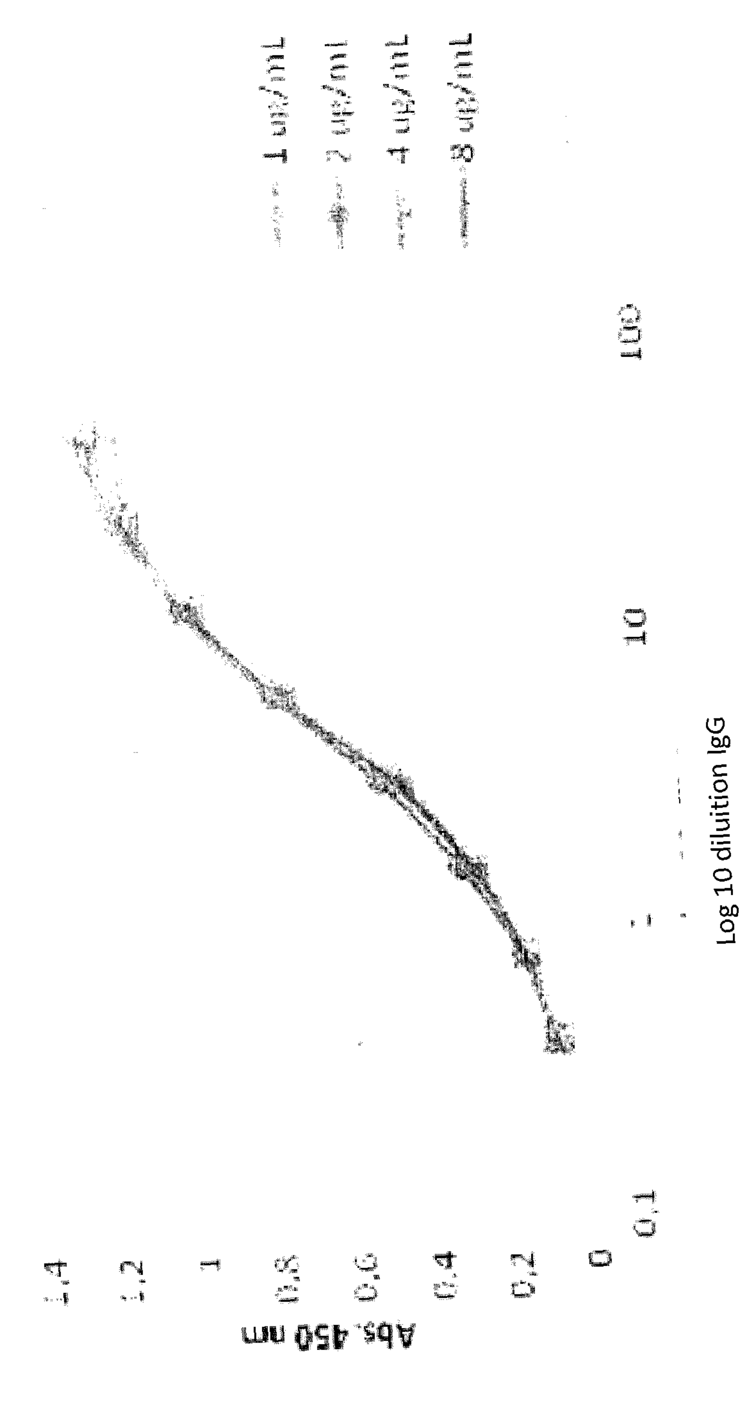

FIG.7

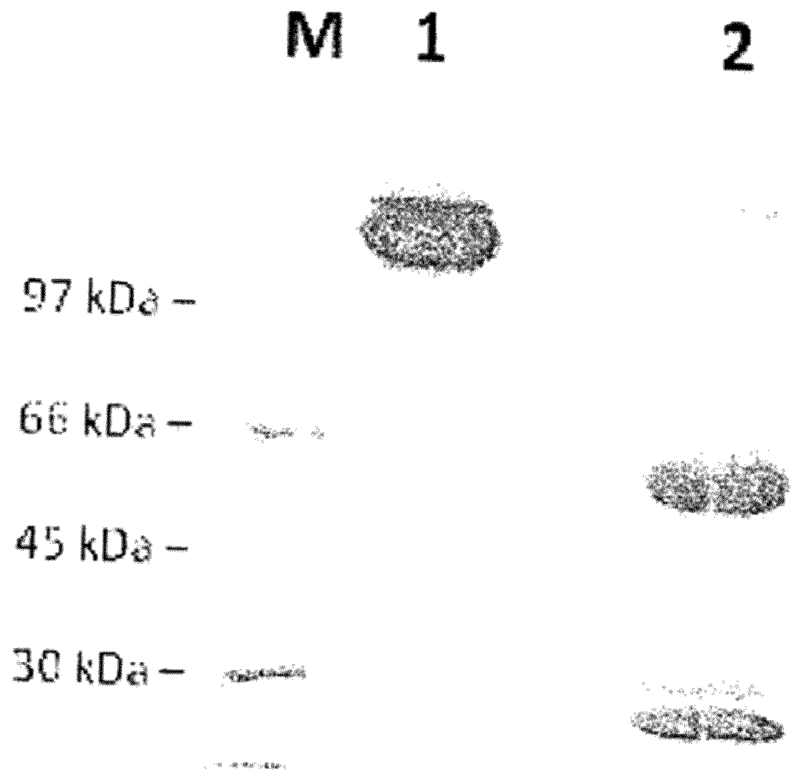

FIG.8

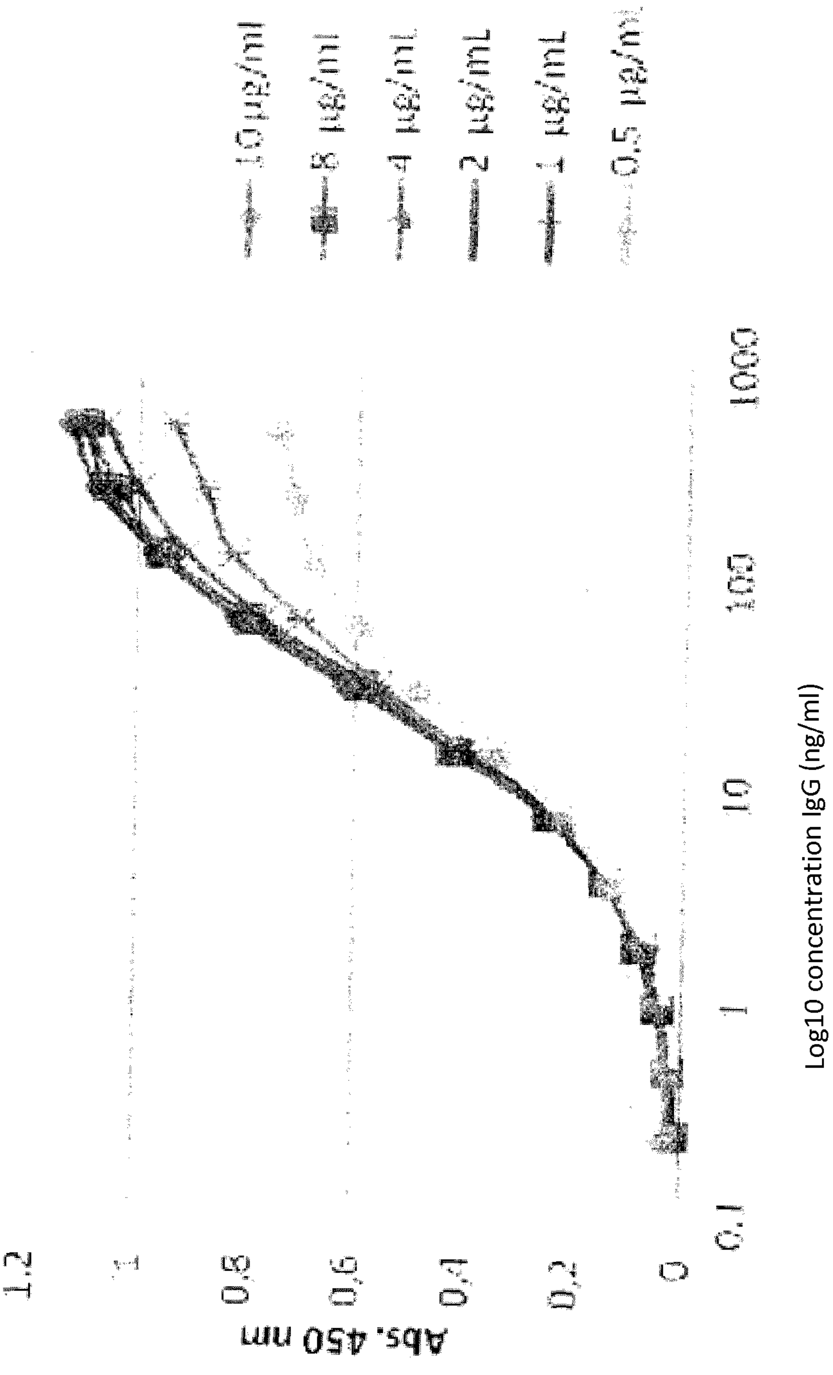
10 µg/ml
8 µg/ml
4 µg/ml
2 µg/mL
1 µg/mL
0.5 µg/ml
1.2
1
0.8
0.6
0.4
0.2
0
Abs. 450 nm
0.1
1
10
100
1000
Log10 concentration IgG (ng/ml)

FIG.9

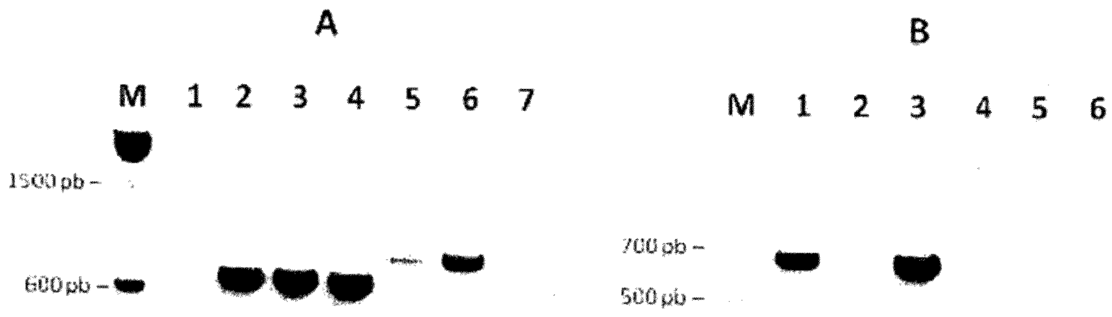

FIG.10

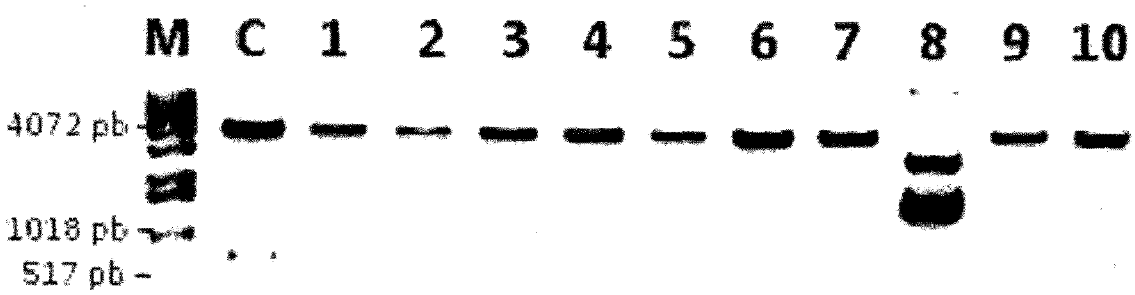

FIG.11

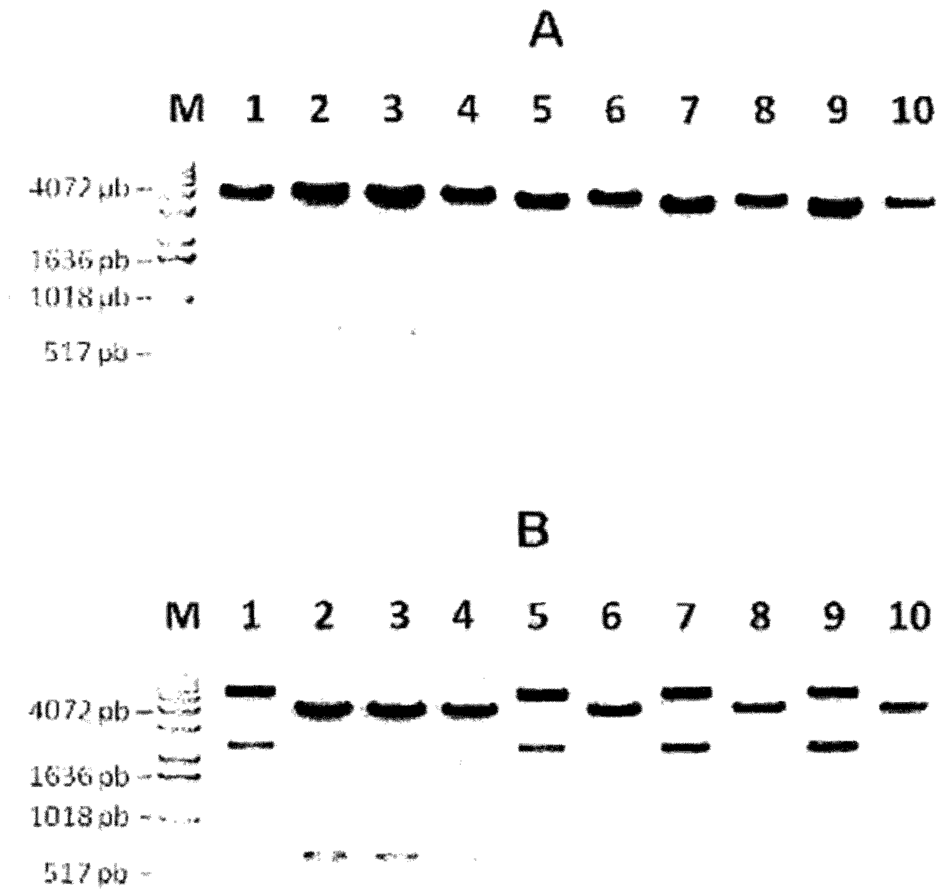
A
M 1 2 3 4 5 6 7 8 9 10
4072 pb
1636 pb
1018 pb
517 pb
B
M 1 2 3 4 5 6 7 8 9 10
4072 pb
1636 pb
1018 pb
517 pb

FIG.12

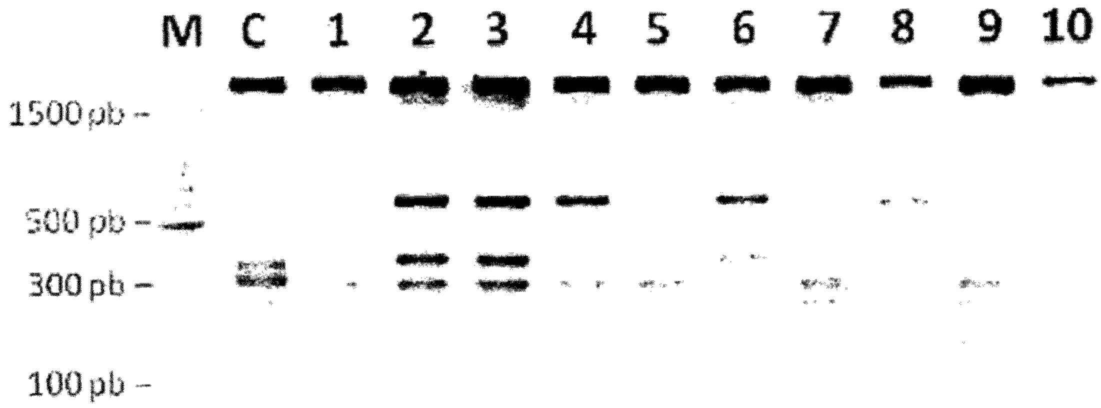

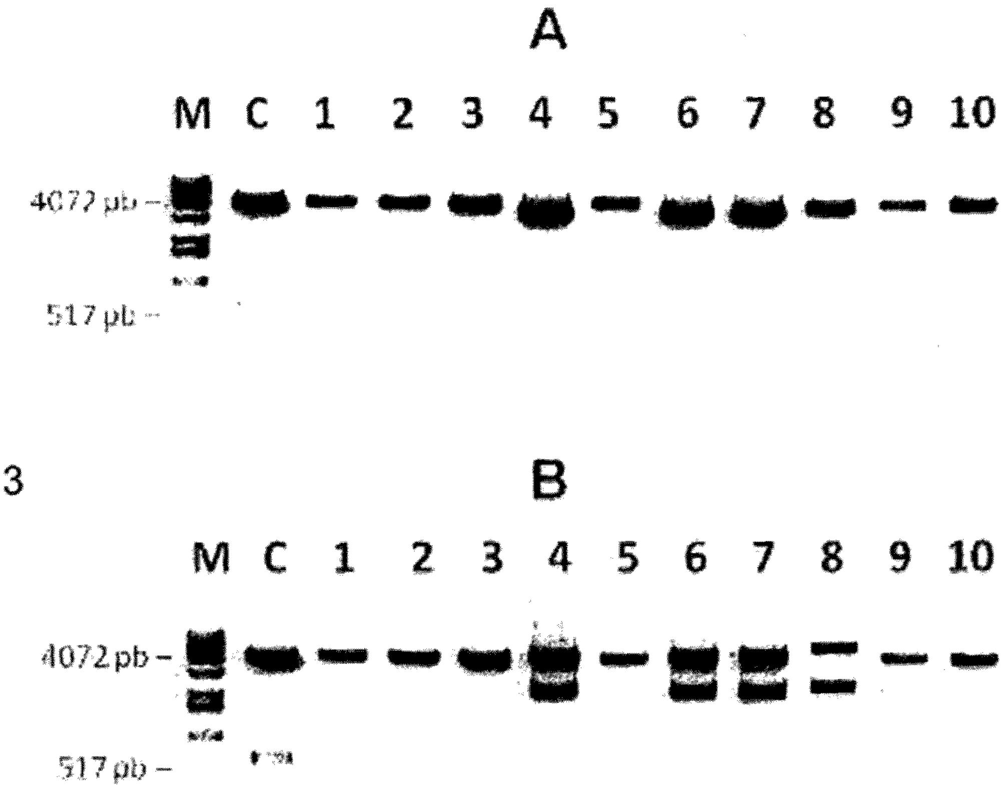
A
M C 1 2 3 4 5 6 7 8 9 10
4072 pb –
517 pb –
B
M C 1 2 3 4 5 6 7 8 9 10
4072 pb –
517 pb –

FIG.13

FIG.14

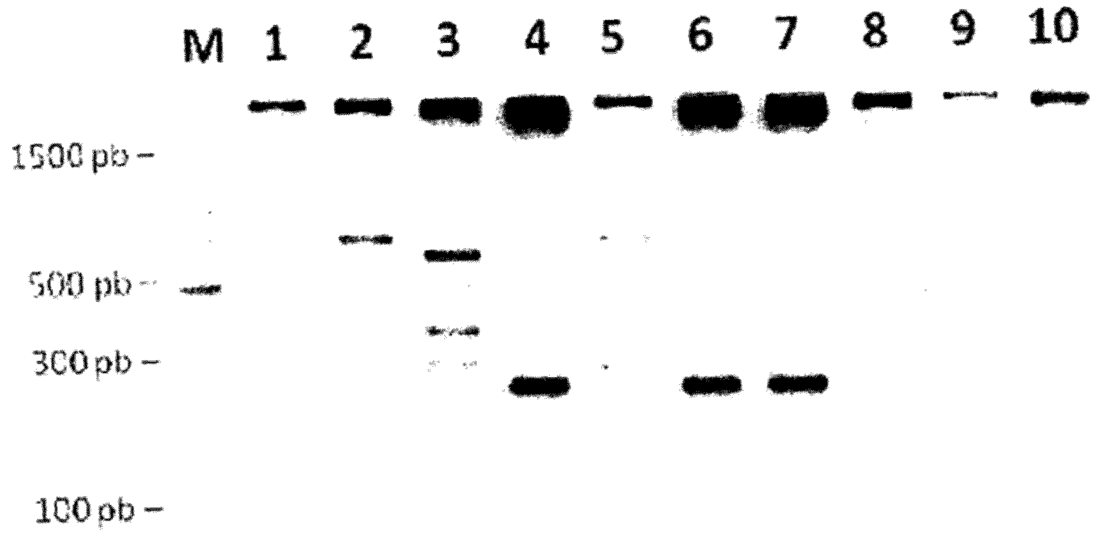

FIG.15

| | FR1 | CDR1 | FR2 | CDR2 | FR3 | CDR3 | FR4 | CL |
|---|---|---|---|---|---|---|---|---|
| Clone 2 | ▪▪◊♠=–▲–♣○–♥=▪– | = | = | ––––○–– | = | = | = | = |
| Clone 10 | ♥▪▪◊♠=–▲–♣○–♥=♦– | = | = | ––––○–– | = | = | = | = |
| Clone 1 | ♠▼=▪♠–▲–◘○–▪▲♥– | = | = | ––––○–– | = | = | = | = |
| Clone 9 | ♠▼=▪♠– –◘○–▪▲♥– | = | = | ––––♣–– | = | = | = | = |
| | : :: * * :: * :: * | | | * * * * : * * | | | | |

FIG.16

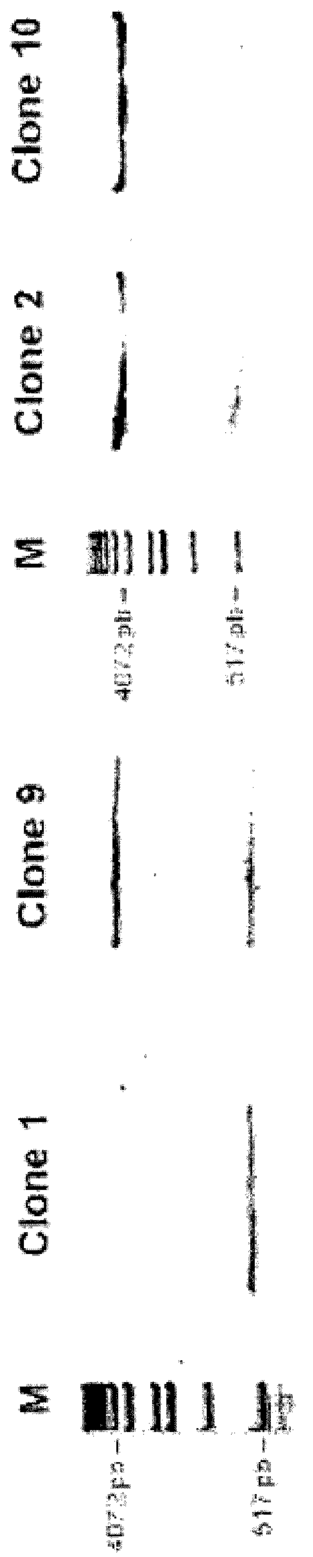

FIG.17

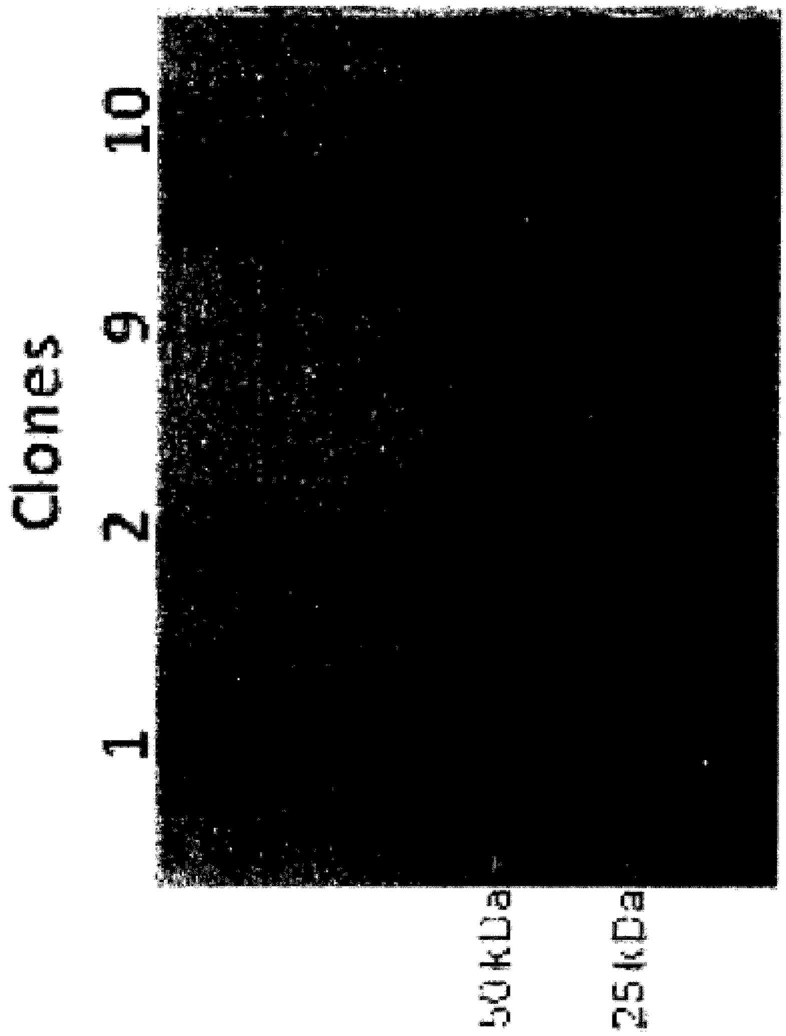

FIG.18

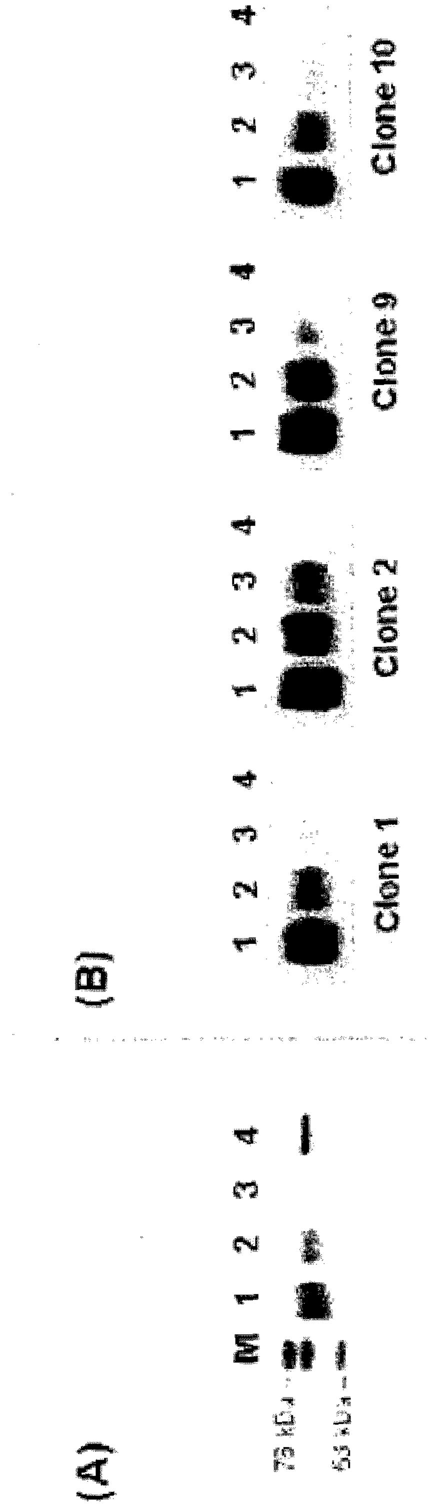

FIG.19

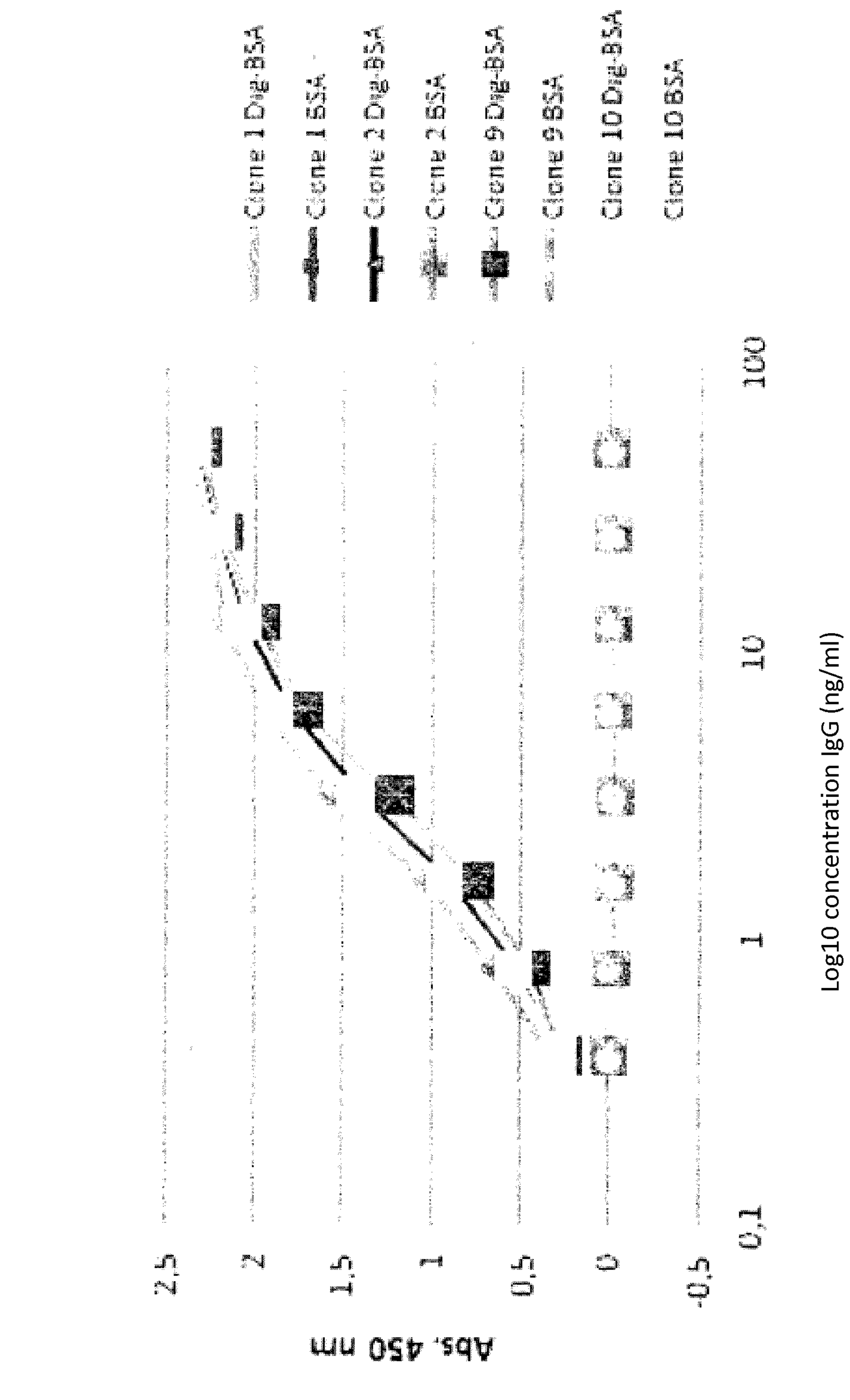
Abs. 450 nm
2,5
2
1,5
1
0,5
0
-0,5
0,1
1
10
100
Log10 concentration IgG (ng/ml)
Clone 1 Dig-BSA
Clone 1 BSA
Clone 2 Dig-BSA
Clone 2 BSA
Clone 9 Dig-BSA
Clone 9 BSA
Clone 10 Dig-BSA
Clone 10 BSA

FIG.20

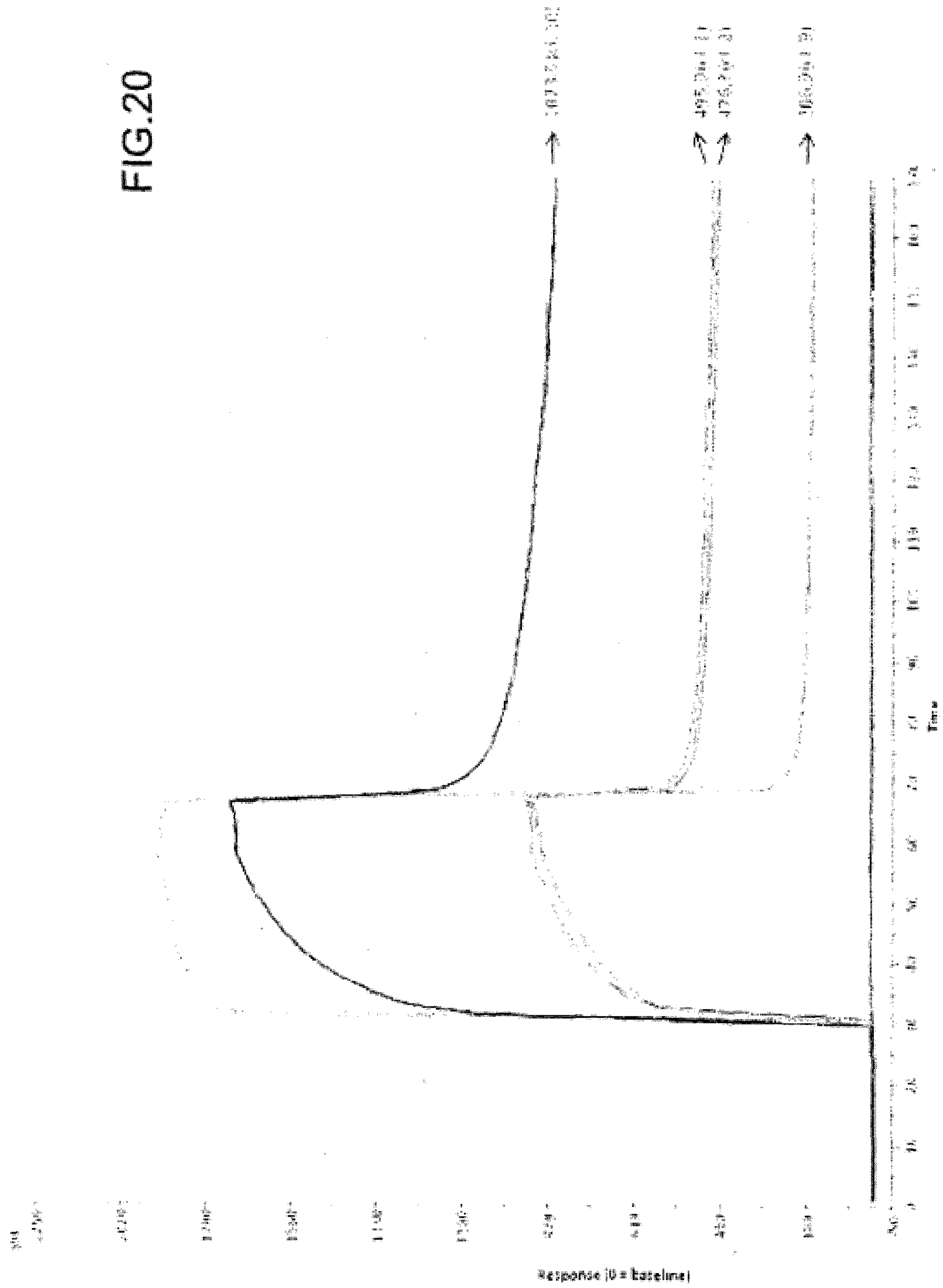

**INTERNATIONAL SEARCH REPORT**

International application No. PCT/BR2013/000164

A. CLASSIFICATION OF SUBJECT MATTER

**C12N 15/13 (2006.01), C07K 16/16 (2006.01), C12N 15/70 (2006.01), G01N 33/53 (2006.01)**

According to International Patent Classification (IPC) or to both national classification and IPC

B. FIELDS SEARCHED

Minimum documentation searched (classification system followed by classification symbols)

**C12N, C07K**

Documentation searched other than minimum documentation to the extent that such documents are included in the fields searched

Electronic data base consulted during the international search (name of data base and, where practicable, search terms used)

**DERWENT, BIOSIS, MEDLINE, SCISEARCH**

C. DOCUMENTS CONSIDERED TO BE RELEVANT

| Category* | Citation of document, with indication, where appropriate, of the relevant passages | Relevant to claim No. |
|---|---|---|
| X | Short, et al., "A single H: CDR3 residue in the anti-digoxin antibody 26-10 modulates specificity for C16-substituted digoxin analogs". Protein Engineering (2001), vol. 14, n°4, pages 287-296. - the whole document - | 1 to 7 |
| A | Ball Jr., et al. "Selection of Peptidic Mimics of Digoxin from Phage-displayed Peptide Libraries by Anti-digoxin Antibodies". J. Mol. Biol. (2000) vol. 301, pages 101–115. - the whole document - | |
| A | US 4803167 B1 07 FEB 1989 (07.02.1989) | |

[X] Further documents are listed in the continuation of Box C. [X] See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 08 JUL 2013 (08.07.2013) | 12 JUL 2013 (12.07.2013) |
| Name and mailing address of the ISA/ INPI | Authorized officer |
| Facsimile No. | Telephone No. |

Form PCT/ISA/210 (second sheet) (July 2009)

**INTERNATIONAL SEARCH REPORT**

International application No.
PCT/BR2013/000164

C (Continuation). DOCUMENTS CONSIDERED TO BE RELEVANT

| Category* | Citation of document, with indication, where appropriate of the relevant passages | Relevant to claim No. |
|---|---|---|
| A | CN 1375505 A (GEN HOSPITAL PLA [CN])<br>23 OCT 2002 (23.10.2002) | |

Form PCT/ISA/210 (continuation of second sheet) (July 2009)

**INTERNATIONAL SEARCH REPORT**
Information on patent family members

International application No.
PCT/BR2013/000164

| | | | |
|---|---|---|---|
| US 4803167 B1 | -- | NONE | |
| CN 1375505 A | 2002-10-23 | CN 1259339 C | 2006-06-14 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4803167 A
- CN 1375505


### Non-patent literature cited in the description


- **ALT3CHUL et al.** *National Center for Biotechnology Information (NCBI), Bethesda, MD, USA,* 1990
- **LARKIN et al.** *European Bioinformatics Institute (EBI) belonging to the European Molecular Biology Laboratory (EMBL),* 2007
- **ABBAS, A. K. ; LICHTMAN, A. H.** Imunologia celular e molecular. Elsevier, 2005, 576
- **AGGARWAL, S.** What's fueling the biotech engine-2009-2010. *Nat. Biotechnol.,* 2010, vol. 28, 1165-1171
- **ALTSCHUL, S. F. ; GISH, W. ; MILLER, W. ; MYERS, E. W. ; LIPMAN, D.** J. Basic local alignment search tool. *J. Mol. Biol.,* 1990, vol. 215, 403-410
- **ANDRÉS, V. L.** G. Revisión sistemática sobre la efectividad e indicaciones de los anticuerpos antidigoxina en la intoxicación digitálica. *Rev Esp Cardiol.,* 2000, vol. 53, 49-58
- **ANT AN, E. M. ; SMITH, T. W.** Digitalis toxicity. *Annu. Rev. Med.,* 1985, vol. 36, 357-367
- **ANTMAN, E. M. ; WENGER, T. L. ; BUTLER, V. P. JR. ; HABER, E. ; SMITH, T. W.** Treatment of 150 cases of life-threatening digitalis intoxication with digoxin-specific Fab antibody fragments. Final report of a multicenter study. *Circulation,* 1990, vol. 81, 1744-1752
- **ARAP, M. A.** Phage display technology - Applications and innovations. *Genet. Mol. Biol.,* 2005, vol. 28, 1-9
- **ARGIRIADI, M. A. ; XIA G, T. ; WU, C ; GHAYUR, T. ; BORHANI, D. W.** Unusual water-mediated antigenic recognition of the proinflammatory cytokine interleukin-18. *J. Biol. Chem.,* 2009, vol. 36, 24478-4489
- **BARBAS, C. F. 3RD. ; KANG, A. S. ; LERNER, R. A. ; BENKOVIC, S. J.** Assembly of combinatorial antibody libraries on phage surfaces: the gene III site. *Proc. Natl. Acad. Sci. U. S. A.,* 1991, vol. 88, 7978-7982
- **BARBAS, C. F. ; BURTON, D. R. ; SCOTT, J. K. ; SILVERMAN, G. J.** Phage display: a laboratory manual. Cold Spring Harbor Laboratory Press, 2001, 736
- **BONG, Y. S. ; CHO, S. H. ; NHAM, S. U. ; LEE, Y. I.** Cloning and characterization of cDNAs coding for heavy and light chains of agglutinating monoclonal antibody (HAG12is1rh) specific for human red blood cells. *Biochim. Biophys. Acta,* 1998, vol. 10, 156-158
- **BUTLER, JR, V. P. ; CHEN, J. P.** Digoxin-specific antibodies. *Proc. Natl. Acad. Sci. U. S. A.,* 1967, vol. 57, 71-78
- **BUTLER, JR, V. P. ; SCHMIDT, D. H. ; SMITH, T. W. ; HABER, E. ; RAYNOR, B. D. ; DEMARTINI, P.** Effects of sheep digoxin- specific antibodies and their Fab fragments on digoxin pharmacokinetics in dogs. *J. Clin. Invest.,* 1977, vol. 59, 345-359
- **CALDAS, C ; COELHO, V ; KALIL, J. ; MORO, A. M. ; MARANHAO, A. Q. ; BRÍGIDO, M. M.** Humanization of the anti-CD18 antibody 6.7: an unexpected effect of a framework residue in binding to antigen. *Mol. Immunol.,* 2003, vol. 39, 941-952
- **CHOMCZYNSKI, P. ; SACCHI, N.** Single-step method of RNA isolation by acid guanidinium thiocyanate-phenol-chloroform extraction. *Anal. Biochem.,* 1987, vol. 162, 156-159
- **CLACKSON, T. ; HOOGENBOOM, H. R. ; GRIFFITHS, A. D. ; WINTER, G.** Making antibody fragments using phage display libraries. *Nature,* 1991, vol. 352, 624-628
- **COSTAGLIOLA S ; BONOMI M ; MORGENTHALER NG ; VAN DURME J ; PANNEELS V ; REFETOFF S ; VASSART G.** Delineation of the discontinuous-conformational epitope of a monoclonal antibody displaying full in vitro and in vivo thyrotropin activity. *Mol Endocrinol.,* 2004, vol. 18, 3020-3034
- **EICHHORN, E. J. ; GHEORGHIADE, M.** *Digoxin. Prog. Cardiovasc. Dis.,* 2002, vol. 44, 251-266
- **ERLANGER, B. F. ; BEISER, S. M.** Antibodies specific for ribonucleosides and ribonucleotides and their reaction with DNA. *Proc. Natl. Acad. Sci. U. S. A.,* 1964, vol. 52, 68-74
- **FLANAGAN, R. J. ; JONES, A. L.** Fab antibody fragments: some applications in clinical toxicology. *Drug Saf.,* 2004, vol. 27, 1115-1133

- **GHEORGHIADE, M. ; VAN VELDHUISEN, D. J. ; COLUCCI, W. S.** Contemporary use of digoxin in the management of cardiovascular disorders. *Circulation,* 2006, vol. 113, 2556-2564
- **GOLINELLI-PIMPANEAU, B. ; GONCALVES, O. ; DINTINGER, T. ; BLANCHARD, D. ; KNOSSOW, M. ; TELLIER, C.** Structural evidence for a programmed general base in the active site of a catalytic antibody. *Proc. Natl. Acad. Sci. U. S. A.,* 2000, vol. 18, 9892-9895
- **GONÇALVES, O. ; DINTINGER, T. ; LEBRETON, J. ; BLANCHARD, D. ; TELLIER, C.** Mechanism of an antibody-catalysed allylic isomerization. *Biochem. J.,* 2000, vol. 3, 691-698
- **HORWITZ AH ; NADELL R ; PREUGSCHAT F ; BETTER M.** Chimeric immunoglobulin light chains are secreted at different levels: influence of framework-1 amino acids. *Mol. Immunol.,* 1994, vol. 31, 683-692
- **HUNTER, M. M. ; MARGOLIES, M. N. ; JU, A. ; HABER, E.** High- affinity monoclonal antibodies to the cardiac glycoside, digoxin. *J. Immunol.,* 1982, vol. 129, 1165-1172
- **HUSE, W. D. ; SASTRY, L. ; IVERSON, S. A. ; KANG, A. S. ; ALTING-MEES, M. ; BURTON, D. R. ; BENKOVIC, S. J. ; LERNER, R. A.** Generation of a large combinatorial library of the immunoglobulin repertoire in phage lambda. *Science,* 1989, vol. 246, 1275-1281
- **KABAT, E. A. ; WU, T. T. ; PERRY, H. M. ; GOTTES AN, K. S. ; FOELLER, C.** Sequences of proteins of immunological interest. MD: National Institute of Health, 2001, 2597
- **KOHLER, G. ; MILSTEIN, C.** Continuous cultures of fused cells secreting antibody of predefined specificity. *Nature,* 1975, vol. 256, 495-497
- **KRYKBAEV, R. A. ; LIU, W. R. ; JEFFREY, P. D. ; MARGOLIES, M. N.** Phage display-selected sequences of the heavy- chain CDR3 loop of the anti-digoxin antibody 26-10 define a high affinity binding site for position 16-substituted analogs of digoxin. *J. Biol. Chem.,* 2001, vol. 276, 8149-8158
- **KRYKBAEV, R. A. ; TSANTILI, P. ; JEFFREY, P. D. ; MARGOLIES, M. N.** Modifying specificity of antidigoxin antibodies using insertional mutagenesis. *Protein Sci.,* 2002, vol. 11, 2899-2908
- **KUBA, H. ; FURUKA A, A. ; OKAJIMA, T. ; FURUKAWA, K.** Efficient bacterial production of functional antibody fragments using a phagemid vector. *Protein Expr. Purif.,* 2008, vol. 58, 292-300
- **KWONG, K. Y. ; RADER, C. E.** coli expression and purification of Fab antibody fragments. *Curr. Protoc. Protein Sci.,* 2009, vol. 55, 6.10.1-6.10.14
- **LAI, Y. S. ; JOHN, J. A. ; GUO, I. C. ; CHEN, S. C. ; FANG, K. ; CHANG, C. Y.** In vitro efficiency of intra- and extracellular immunization with mouse anti-YGNNV antibody against yellow grouper nervous necrosis virus. *Vaccine,* 2002, vol. 20, 3221-3229
- **LAPOSTOLLE, F. ; BORRON, S. W. ; VERDIE, C. ; TABOULET, P. ; GUERRIER, G. ; ADNET, F. ; CLE ESSY, J. L. ; BISMUTH, C. ; BAUD, F. J.** Digoxin-specific Fab fragments as single first- line therapy in digitalis poisoning. *Crit. Care Med.,* 2008, vol. 36, 3014-3018
- **LARKIN, M. A. ; BLACKSHIELDS, G. ; BROWN, N. P. ; CHENNA, R. ; CGETTIGAN, P. A. ; MCWILLIAM, H. ; VALENTIN, F. ; WALLACE, I. M. ; WILM, A. ; LOPEZ, R.** ClustalW and ClustalX version 2.0. *Bioinfomatics,* 2007, vol. 23, 2947-2948
- **LEVY, R. ; MOLINEUX, I. J. ; IVERSON, B. L. ; GEORGIOU, G.** Isolation of trans-acting genes that enhance soluble expression of scFv antibodies in the E. coli cytoplasm by lambda phage display. *J Immunol. Meth.,* 2007, vol. 321, 164-173
- **MARKS, J. D. ; HOOGENBOOM, H. R. ; BONNERT, T. P. ; MCCAFFERTY, J. ; GRIFFITHS, A. D. ; WINTER, G.** By-passing immunization. Human antibodies from V-gene libraries displayed on phage. *J. Mol. Biol.,* 1991, vol. 222, 581-597
- **MCCAFFERTY, J. ; GRIFFITHS, A. D. ; WINTER, G. ; CHISWELL, D. J.** Phage antibodies: filamentous phage displaying antibody variable domains. *Nature,* 1990, vol. 348, 552-554
- **MORO, A. M. ; RODRIGUES, M. T. A.** Anticorpos monoclonais para a clinica. *Biotecnologia Ciência e Desenvolvimento,* 2001, vol. 22, 32-35
- **MUDGETT-HUNTER, M. ; ANDERSON, W. ; HABER, E. ; MARGOLIES, M. N.** Binding and structural diversity among high-affinity monoclonal anti-digoxin antibodies. *Mol. Immunol.,* 1985, vol. 22, 477-488
- **ORLANDI, R. ; GUSSOW, D. H. ; JONES, P. T. ; WINTER, G.** Cloning immunoglobulin variable domains for expression by the polymerase chain reaction. *Proc. Natl. Acad. Sci. U. S. A.,* 1989, vol. 86, 3833-3837
- **PANKA, D. J. ; MUDGETT-HUNTER,. ; PARKS, D. R. ; PETERSON, L. L. ; HERZENBERG, L. A. ; HABER, E. ; MARGOLIES, M. N.** Variable region framework differences result in decreased or increased affinity of variant anti-digoxin antibodies. *Proc. Natl. Acad. Sci. U. S. A.,* 1988, vol. 85, 3080-3084
- **PAULA, F. J.** DE. Fração sérica de pacientes urêmicos expandidos com atividade digoxina-símile inibidora da Na+K+ ATPase: isolamento, efeitos biológicos e caracterização com anticorpos monoclonais. *Tese (Doutorado em Nefrologia) - Faculdade de Medicina, Universidade de São Paulo, São Paulo,* 1993, 152 f
- **RINDERKNECHT, M. ; VILLA, A. ; BALLMER-HOFER, K. ; NERI, D. ; DETMAR, M.** Phage-derived fully human monoclonal antibody fragments to human vascular endothelial growth factor-C block its interaction with VEGF receptor-2 and 3. *PLoS One.,* 2010, vol. 5, e11941

- **SCHASFOORT, R. B. M. ; TUDOS, A. J.** Handbook of surface plasmon resonance. Royal Society of Chemistry Publishing, 2008, 403
- **SCHILDBACH, J. F ; PANKA, D. J. ; PARKS, D. R. ; JAGER, G. C ; NOVOTNY, J. ; HERZENBERG, L. A. ; MUDGETT-HUNTER, M. ; BRUCCOLERI, R. E. ; HABER, E. ; MARGOLIES, M. N.** Altered hapten recognition by two anti-digoxin hybridoma variants due to variable region point mutations. *J. Biol. Chem.,* 1991, vol. 266, 4640-4647
- **SCHILDBACH, J. F. ; NEAR, R. I. ; BRUCCOLERI, R. E. ; HABER, E. ; JEFFREY, P. D. ; NG, S. C. ; NOVOTNY, J. ; SHERIFF, S. ; MARGOLIES, M. N.** Heavy chain position 50 is a determinant of affinity and specificity for the anti-digoxin antibody 26-10. *J. Biol. Chem.,* 1993, vol. 268, 21739-21747
- **SHORT, M. K. ; JEFFREY, P. D. ; KWONG, R. F ; MARGOLIES, M. N.** Contribution of antibody heavy chain CDR1 to digoxin binding analyzed by random mutagenesis of phage-displayed Fab 26-10. *J. Biol. Chem.,* 1995, vol. 270, 28541-28550
- **SHORT, M. K. ; KRYKBAEV, R. A. ; JEFFREY, P. D. ; MARGOLIES, M. N.** Complementary combining site contact residue mutations of the anti-digoxin Fab 26-10 permit high affinity wild-type binding. *J. Biol. Chem.,* 2002, vol. 277, 16365-16370
- **SMITH, G. P.** Filamentous fusion phage: novel expression vectors that display cloned antigens on the virion surface. *Science,* 1985, vol. 228, 1315-1317
- **SMITH, T. W. ; BUTLER, V. P. JR. ; HABER, E.** Characterization of antibodies of high affinity and specificity for the digitalis glycoside digoxin. *Biochemistry,* 1970, vol. 9, 331-337
- **SMITH, T. W. ; HABER, E. ; YETMAN, L. ; BUTLER, JR.** Reversal of advanced digoxin intoxication with Fab fragments of digoxin-specific antibodies. *N. Eng J. Med.,* 1976, vol. 294, 797-800
- **STRACHAN, G. ; MCELHINEY, J. ; DREVER, MR. ; MCINTOSH, F. ; LAWTO, L. A. ; PORTER, A. J.** Rapid selection of anti-hapten antibodies isolated from synthetic and semi-synthetic antibody phage display libraries expressed in Escherichia coli. *FEMS Microbiol Lett.,* 2002, vol. 210, 257-261
- **TONEGAWA, S.** Somatic generation of antibody diversity. *Nature,* 1983, vol. 302, 575-581
- **TSURUTA, L. R. ; TOMIOKA, Y. ; HISHINUMA, T. ; KATO, Y. ; ITOH, K. ; SUZUKI, T. ; GGURI, H. ; HIRAMA,. ; GOTO, J. ; MIZUGAKI, M.** Characterization of 11-dehydro-thromboxane B2 recombinant antibody obtained by phage display technology. *Prostaglandins Leukot Essent Fatty Acids,* 2003, vol. 68, 273-284
- An account of the foxglove and some of its medicai uses; with practical remarks on the dropsy, and some other diseases. **WITHERING, W.** Classics of Cardiology. Henry Schyuman, Dover Publications, 1941, vol. 1, 231-252